(19) Europäisches Patentamt
European Patent Office
Office européen des brevets

(11) Numéro de publication: **0 580 713 B1**

(12) # FASCICULE DE BREVET EUROPEEN

(45) Date de publication de fascicule du brevet: **01.03.95**

(51) Int. Cl.$^6$: **A61K 7/13**

(21) Numéro de dépôt: **92909472.0**

(22) Date de dépôt: **30.03.92**

(86) Numéro de dépôt internationale :
**PCT/FR92/00289**

(87) Numéro de publication internationale :
**WO 92/18093 (29.10.92 92/27)**

(54) **PROCEDE DE TEINTURE DES FIBRES KERATINIOUES AVEC DES AMINOINDOLES, A pH BASIOUE, COMPOSITIONS MISES EN OEUVRE.**

(30) Priorité: **18.04.91 FR 9104801**

(43) Date de publication de la demande:
**02.02.94 Bulletin 94/05**

(45) Mention de la délivrance du brevet:
**01.03.95 Bulletin 95/09**

(84) Etats contractants désignés:
**AT BE CH DE DK ES FR GB GR IT LI NL SE**

(56) Documents cités:
**EP-A- 0 271 186** **EP-A- 0 348 280**
**EP-A- 0 414 585** **EP-A- 0 424 261**
**EP-A- 0 425 345** **EP-A- 0 462 883**
**EP-A- 0 465 339** **EP-A- 0 465 340**
**FR-A- 2 626 771** **GB-A- 2 205 329**
**US-A- 4 013 404**

(73) Titulaire: **L'OREAL**
**14, Rue Royale**
**F-75008 Paris (FR)**

(72) Inventeur: **LANG, Gérard**
**44, avenue Lacour**
**F-95210 Saint-Gratien (FR)**
Inventeur: **JUNINO, Alex**
**16, rue Docteur-Bergonié**
**F-93180 Livry-Gargan (FR)**
Inventeur: **COTTERET, Jean**
**15, allée des Meuniers**
**F-78480 Verneuil-sur-Seine (FR)**
Inventeur: **LAGRANGE, Alain**
**5 rue de Montry**
**F-77700 Coupvray (FR)**

(74) Mandataire: **Casalonga, Axel**
**BUREAU D.A. CASALONGA - JOSSE**
**Morassistrasse 8**
**D-80469 München (DE)**

EP 0 580 713 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à un nouveau procédé de teinture des fibres kératiniques, en particulier des fibres kératiniques humaines, mettant en oeuvre des aminoindoles en association avec des bases d'oxydation et un agent oxydant en milieu basique, aux compositions mises en oeuvre au cours de ce procédé et à de nouveaux composés aminoindoles.

Il est connu de teindre les fibres kératiniques et en particulier les cheveux humains avec des compositions tinctoriales contenant en milieu alcalin des précurseurs de colorants d'oxydation et en particulier des p-phénylènediamines des ortho ou para-aminophénols appelés généralement "bases d'oxydation".

On sait également que l'on peut faire varier les nuances obtenues avec ces bases d'oxydation en les associant à des coupleurs encore appelés modificateurs de coloration, choisis notamment parmi les métadiamines aromatiques, les méta-aminophénols et les métadiphénols.

La demanderesse vient de découvrir de façon surprenante que l'utilisation de certains composés du type aminoindole associés à des bases d'oxydation, lorsque cette association était appliquée en présence d'un agent oxydant et à pH basique sur les cheveux, conduisait à des colorations présentant une excellente puissance tinctoriale.

Les colorations ainsi obtenues présentent une excellente ténacité à la lumière, aux lavages, à la transpiration et aux intempéries.

La présente invention a donc pour objet un procédé de teinture des fibres kératiniques, en particulier des fibres kératiniques humaines telles que les cheveux, comprenant l'application sur ces fibres d'une composition contenant au moins un aminoindole de formule (I) telle que définie ci-après, d'un précurseur de colorant d'oxydation encore appelé base d'oxydation et d'un agent oxydant, à pH basique.

L'invention a également pour objet un agent de teinture à deux composants dont l'un des composants comprend l'aminoindole et le précurseur du colorant d'oxydation et l'autre l'agent oxydant, en des quantités telles que le mélange présente un pH basique.

Un autre objet consiste également en des compositions de teinture d'oxydation contenant un précurseur de colorant d'oxydation et l'un de ces composés particuliers comme coupleur.

L'invention a également pour objet la composition prête à l'emploi, contenant les différents agents utilisés pour la teinture, en milieu basique, des cheveux.

D'autres objets de l'invention apparaîtront à la lecture de la description et des exemples qui suivent.

Le procédé de teinture des fibres kératiniques et en particulier des fibres kératiniques humaines telles que les cheveux, conforme à l'invention, est essentiellement caractérisé par le fait que l'on applique sur ces fibres une composition contenant dans un milieu approprié pour la teinture, au moins un coupleur répondant à la formule :

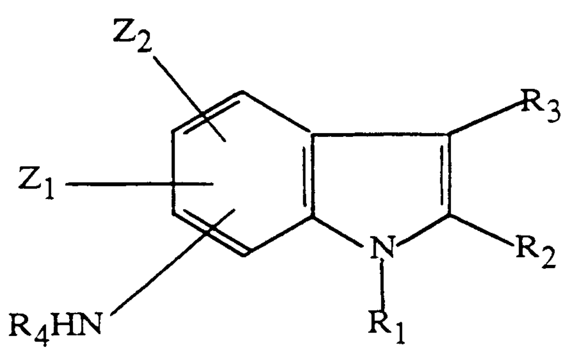

(I)

dans laquelle :

$R_1$ désigne hydrogène ou un groupement alkyle en $C_1$-$C_4$ éventuellement ramifié;

$R_2$ et $R_3$, indépendamment l'un de l'autre, désignent hydrogène ou un groupement alkyle en $C_1$-$C_4$ ou un groupement COOR', R' étant un radical alkyle en $C_1$-$C_4$ ou un atome d'hydrogène; au moins un des groupements $R_2$ et $R_3$ représente un atome d'hydrogène;

$R_4$ représente un atome d'hydrogène ou un groupement alkyle en $C_1$-$C_4$ ou un groupement hydroxyalkyle en $C_1$-$C_4$, polyhydroxyalkyle en $C_2$-$C_4$ ou aminoalkyle en $C_1$-$C_6$, dont l'amine peut éventuellement être mono- ou disubstituée par un alkyle en $C_1$-$C_4$;

le groupement $-NHR_4$ pouvant occuper les positions 4, 6 ou 7;

$Z_1$, $Z_2$, identiques ou différents, représentent un atome d'hydrogène ou un groupement alkyle en $C_1$-$C_4$, un hydroxyle, un halogène ou un alcoxy en $C_1$-$C_4$;

au moins un des groupements $Z_1$ ou $Z_2$ est différent d'hydrogène;

et les sels de tous ces composés;

- au moins un précurseur de colorant d'oxydation;
- au moins un agent oxydant;

le pH de la composition appliquée sur les fibres étant supérieur à 7.

Les composés préférés répondant à la formule (I), utilisés conformément à l'invention, sont les suivants :

(1) le 4-méthyl 6-aminoindole,
(2) le 5-méthyl 6-aminoindole,
(3) le 7-méthyl 4-aminoindole,
(4) le 3-méthyl 7-éthyl 6-aminoindole,
(5) le 2-méthyl 5-hydroxy 6-aminoindole,
(6) le 5,7-diméthyl 6-aminoindole,
(7) le 5,7-diéthyl 6-aminoindole,
(8) le 2-éthoxycarbonyl 5-méthyl 7-aminoindole,
(9) le 2-éthoxycarbonyl 5-chloro 7-aminoindole,
(10) le 2-éthoxycarbonyl 5-éthoxy 7-aminoindole,
(11) le 2-éthoxycarbonyl 5-méthoxy 7-aminoindole,
(12) le 5-méthoxy 7-(4'-diméthylamino 1'-méthylbutyl) aminoindole,
(13) le 5-méthoxy 7-(4'-diméthylaminobutyl)aminoindole,
(14) le 5-méthoxy 7-(4'-diéthylamino 1'-méthylbutyl)aminoindole,
(15) le 5-fluoro 6-aminoindole,
(16) le 5-fluoro 1-sec-butyl 6-aminoindole,
(17) le 5-fluoro 1-n-propyl 6-aminoindole,
(18) le 1-méthyl 2-méthoxycarbonyl 5-méthoxy 6-aminoindole,
(19) le 2-méthoxycarbonyl 5-méthoxy 6-aminoindole,
(20) le 2-éthoxycarbonyl 5-méthoxy 6-aminoindole,
(21) le 2-carboxy 5-méthoxy 6-aminoindole,
(22) le 1,2-diméthyl 5-hydroxy 6-aminoindole,
(23) le 2-méthoxycarbonyl 4-méthoxy 6-aminoindole,
(24) le 7-éthyl 4-aminoindole,
(25) le 7-éthyl 6-aminoindole,
(26) le 7-éthyl 6-N,$\beta$-hydroxyéthylaminoindole.

Les sels sont choisis plus particulièrement parmi les chlorhydrates ou bromhydrates.

Les composés (1) à (7) et (24) à (26) peuvent être préparés par réduction des dérivés nitrés correspondants. Certains de ces dérivés nitrés sont connus d'après l'article BERGMAN & SAND, Tetrahedron, Vol. 46, N° 17, pages 6085 à 6112, 1990. Les composés nitrés non décrits dans cet article peuvent être obtenus selon le même procédé, c'est-à-dire par action d'un trialkylorthoformiate sur une dialkyl ou trialkyl méta- nitroaniline (l'un des groupements alkyle étant situé en position ortho du groupement amino et para du groupement nitro), en présence d'acide p-toluènesulfonique, puis cyclisation du produit obtenu.

Le 4-méthyl 6-nitroindole et le 5-méthyl 6-nitroindole sont nouveaux.

La réduction du groupement nitro s'effectue selon les procédés classiques de réduction, comme par exemple par réduction avec le fer dans l'acide acétique ou avec le zinc en présence d'alcool et de chlorure d'ammonium, ou bien par hydrogénation catalytique sous pression d'hydrogène en présence d'un catalyseur d'hydrogénation.

La réduction peut également se faire par transfert d'hydrogène avec un agent de transfert tel que le cyclohexène en présence d'un catalyseur d'hydrogénation.

Le catalyseur d'hydrogénation peut être constitué par exemple par du palladium ou du rhodium sur un support, tel que du charbon.

Dans le cas où $Z_1$ ou $Z_2$ = Cl, la réduction se fait de préférence avec le fer dans l'acide acétique ou avec le zinc en présence d'alcool et de chlorure d'ammonium.

Les composés de formule (I) dans lesquels $R_4$ est différent d'un atome d'hydrogène, peuvent être préparés selon le procédé suivant.

Le composé (I) peut être obtenu à partir du composé ($R_4$ = H) par les méthodes de substitution des amines aromatiques, selon le schéma réactionnel:

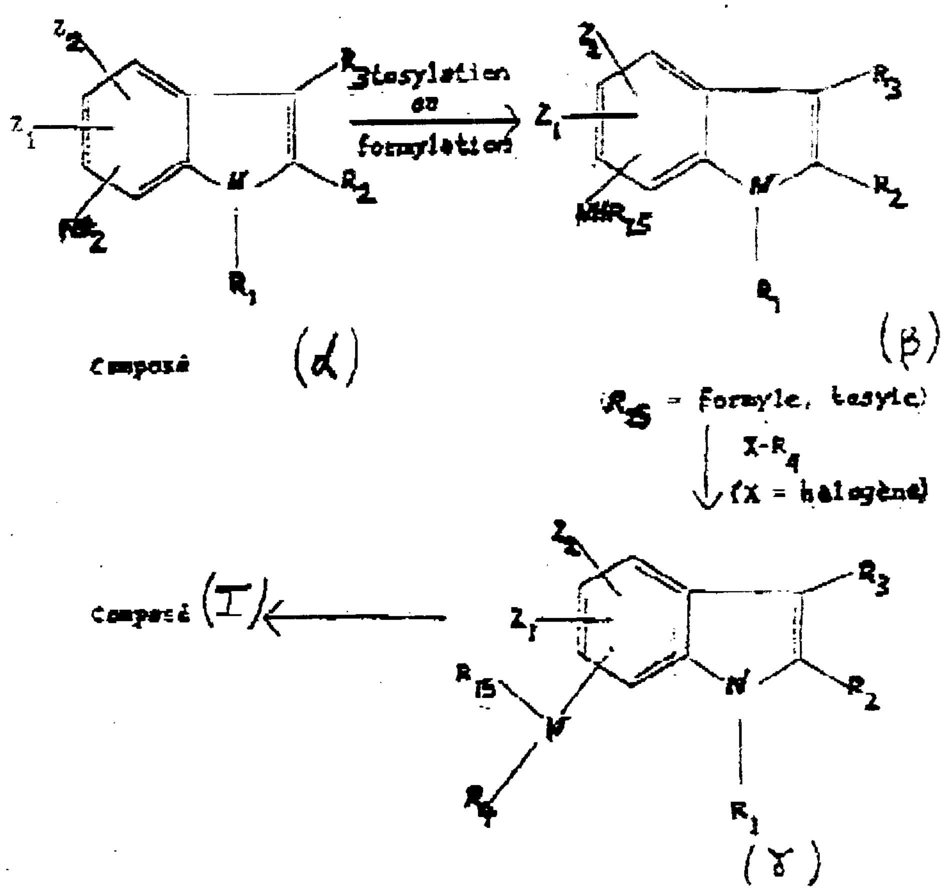


Par formylation ou tosylation, on obtient le composé (β). Le composé (β) est alkylé dans un deuxième temps par l'halogénure d'alkyle X-$R_4$ pour obtenir le composé (γ).

Le produit attendu (I) est obtenu par déformylation ou détosylation du composé (β), selon les méthodes conventionnelles.

Le composé (I) peut également être obtenu, lorsque $R_4$ désigne un groupement alkyle, par réduction du composé (δ) par un hydrure métallique tel que le bromhydrure d'un métal alcalin en présence ou non d'un acide de Lewis tel que l'éthérate de trifluorure de bore.

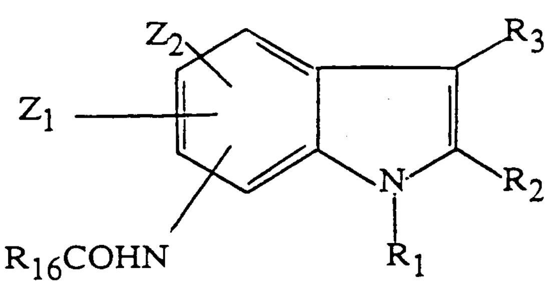

(δ)

formule dans laquelle $R_{16}$ représente un groupement alkyle en $C_1$-$C_3$ ou un hydrogène.

Le composé (δ) étant obtenu à partir du composé (α) selon les procédés connus d'obtention des groupements alkylcarbonylamino.

Parmi les méthodes d'hydroxyalkylation, on peut citer l'action du chloroformiate de β-chloralkyle sur le composé (α) qui permet d'obtenir dans un premier temps le carbamate de β-chloroalkyle correspondant qui, soumis dans un deuxième temps à l'action d'une base minérale forte, permet d'obtenir le composé (I) pour lequel le radical $R_4$ est un radical β-hydroxyalkyle.

Les précurseurs de colorants d'oxydation ou bases d'oxydation sont des composés connus qui ne sont pas des colorants en eux-mêmes et qui forment un colorant par un processus de condensation oxydative, soit sur eux-mêmes, soit en présence d'un coupleur ou modificateur. Ces composés comportent généralement un noyau aromatique portant des groupements fonctionnels, constitués : soit par deux groupements amino; soit par un groupement amino et un groupement hydroxyle; ces groupements étant en position para ou ortho, l'un par rapport à l'autre.

Les précurseurs de colorants d'oxydation de type para, utilisés conformément à l'invention, sont choisis parmi les paraphénylènediamines, les bases dites "doubles", les para-amino phénols, les précurseurs hétérocycliques para, comme la 2,5-diamino pyridine, la 2-hydroxy 5-aminopyridine, la 2,4,5,6-tétraaminopyrimidine.

Parmi les paraphénylènediamines, on peut citer les composés répondant à la formule (II) :

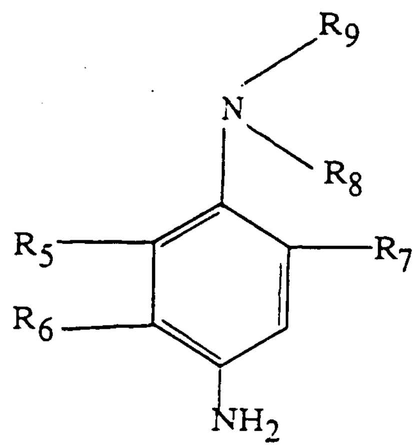

(II)

dans laquelle :

$R_5$, $R_6$ et $R_7$, identiques ou différents, représentent un atome d'hydrogène ou d'halogène, un radical alkyle ayant de 1 à 4 atomes de carbone, un radical alcoxy ayant de 1 à 4 atomes de carbone, un radical carboxy, sulfo, hydroxyalkyle ayant de 1 à 4 atomes de carbone;

$R_8$ et $R_9$, identiques ou différents, représentent un atome d'hydrogène, un radical alkyle, hydroxyalkyle, alcoxyalkyle, carbamyl alkyle, aminoalkyle, mésylaminoalkyle, acétylaminoalkyle, uréidoalkyle, carbalcoxyaminoalkyle, pipéridinoalkyle, morpholinoalkyle; ces groupes alkyle ou alcoxy ayant de 1 à 4 atomes de carbone, ou bien $R_8$ et $R_9$ forment, conjointement avec l'atome d'azote auquel ils sont liés, un hétérocycle pipéridino ou morpholino, sous réserve que $R_5$ ou $R_7$ représente un atome d'hydrogène lorsque $R_8$ et $R_9$ ne représentent pas un atome d'hydrogène, ainsi que les sels de ces composés.

Parmi les composés particulièrement préférés répondant à la formule (II), on peut citer la p-phénylènediamine, la 2-méthyl-p-phénylènediamine, la méthoxyparaphénylènediamine, la chloroparaphénylènediamine, la 2,6-diméthylparaphénylènediamine, la 2,5-diméthylparaphénylènediamine, la 2,3-diméthylparaphénylènediamine, la 2,6-diéthylparaphénylènediamine, la 2-méthyl 5-méthoxyparaphénylènediamine, la 2,6-diméthyl 5-méthoxyparaphénylènediamine, la N,N-diméthylparaphénylène diamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl) aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl) aniline, la 3-méthyl 4-amino N,N-(éthyl,β-sulfoéthyl)aniline, la N-[(4'-amino)phényl]morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, la 2-hydroxyméthylparaphénylènediamine, la N,N-diméthyl 3-méthyl paraphénylènediamine, la N,N(éthyl,β-hydroxyéthyl)-paraphénylène diamine, la N-(dihydroxypropyl)paraphénylènediamine, la N-4'-aminophényl p-phénylènediamine, la N-phényl p-phénylènediamine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Les bases dites doubles sont des bis-phénylalkylènediamines, répondant à la formule :

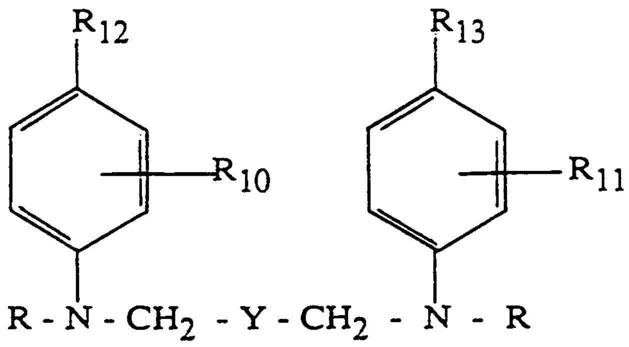

(III)

dans laquelle :

$R_{12}$ et $R_{13}$, identiques ou différents, représentent des groupements hydroxyle ou $NHR_{14}$, où $R_{14}$ désigne un atome d'hydrogène ou un radical alkyle inférieur;

$R_{10}$ et $R_{11}$, identiques ou différents, représentent soit des atomes d'hydrogène, soit des atomes d'halogène, soit encore des groupements alkyle;

R représente un atome d'hydrogène, un groupe alkyle, hydroxyalkyle ou aminoalkyle, dont le reste amino peut être substitué;

Y représente un radical pris dans le groupe constitué par les radicaux suivants :

$-(CH_2)_n-$,$(CH_2)_{n'}-O-(CH_2)_{n'}-$,

$(CH_2)_{n'}-CH\ OH-(CH_2)_{n'}$;

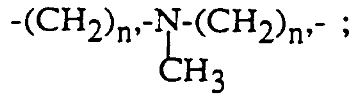

n étant un nombre entier compris entre 0 et 8 et n' un nombre entier compris entre 0 et 4, cette base pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (III), on peut citer le N,N'bis-(β-hydroxyéthyl)N,N'-bis(4'-aminophényl)-1,3-diamino 2-propanol, la N,N'bis-(β-hydroxyéthyl)N,N'-bis(4'-aminophényl)éthylènediamine, la N,N'bis-(4-aminophényl)tétraméthylènediamine, la N,N'bis-(β-hydroxy éthyl)N,N'bis(4-aminophényl)-tétraméthylènediamine, la N,N'bis-(4-méthylaminophényl)tétraméthylènediamine, la N,N'bis(éthyl)N,N'-bis (4'-amino 3'-méthylphényl)éthylènediamine.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-amino phénol, le 2-hydroxyméthyl 4-aminophénol, le 2-(β-hydroxyéthyl) 4-aminophénol, le 2-méthoxy 4-aminophénol, le 3-méthoxy 4-amino phénol, le 2,5-diméthyl 4-aminophénol, le 2-méthoxyméthyl 4-amino phénol, le 2-éthoxyméthyl 4-aminophénol, le 2-β-hydroxyéthoxyméthyl 4-aminophénol.

Les précurseurs de colorants d'oxydation de type ortho sont choisis parmi les orthoaminophénols, comme le 1-amino 2-hydroxy benzène, le 6-méthyl 1-hydroxy 2-aminobenzène, le 4-méthyl 1-amino 2-hydroxybenzène, le 4-acétylamino 1-amino 2-hydroxybenzène et les orthophénylènediamines.

L'agent oxydant est choisi de préférence parmi le peroxyde d'hydrogène, le peroxyde durée, les bromates de métaux alcalins, les persels tels que les perborates et les persulfates. Le peroxyde d'hydrogène est particulièrement préféré.

Le pH de la composition appliquée sur les fibres kératiniques, en particulier les cheveux, a une valeur supérieure à 7 et est compris de préférence entre 8 et 11. Ce pH est ajusté par l'utilisation d'agents alcalinisants bien connus dans le domaine de la teinture des fibres kératiniques, et en particulier des cheveux humains, tels que l'ammoniaque, les carbonates alcalins, les alcanolamines comme la mono-, la di- ou la triéthanolamine, ainsi que leurs dérivés ou les hydroxydes de sodium ou de potassium.

Les composés de formule (I) sont présents dans la composition appliquée sur les fibres kératiniques, dans des proportions comprises de préférence entre 0,01 et 3,5% en poids par rapport au poids total de la composition.

Les compositions, définies ci-dessus, appliquées dans la teinture des fibres kératiniques, peuvent également contenir en plus des coupleurs hétérocycliques de formule (I), d'autres coupleurs connus en eux-mêmes tels que des métadiphénols, des métaaminophénols, des métaphénylènediamines, des méta N-acylaminophénols, des métauréidophénols, des métacarbalcoxyaminophénols, l'$\alpha$-naphtol, des coupleurs possédant un groupement méthylène actif, tels que les composés dicétoniques, les pyrazolones, les coupleurs hétérocycliques ou le 4-hydroxyindole, le 6-hydroxyindole ou le 7-hydroxyindole.

Parmi ces coupleurs pouvant être utilisés en plus des coupleurs de formule (I), on peut citer le 2,4-dihydroxyphénoxyéthanol, le 2,4-dihydroxyanisole, le métaaminophénol, la résorcine, le monométhyl éther de résorcine, la 2-méthylrésorcine, le pyrocatéchol, le 2-méthyl 5-N-($\beta$-hydroxyéthyl)aminophénol, le 2-méthyl 5-N-($\beta$-mésylamino éthyl)aminophénol, la 6-hydroxybenzomorpholine, le 2,4-diamino anisole, le 2,4-diaminophénoxyéthanol, la 6-aminobenzomorpholine, le [2-N-($\beta$-hydroxyéthyl)amino 4-amino]-phénoxyéthanol, le 2-amino 4-N-($\beta$-hydroxyéthyl)aminoanisole, le (2,4-diamino)phényl-$\beta$,$\gamma$-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol, le 3,4-méthylènedioxyphénol, la 3,4-méthylènedioxy aniline, la 2-chlororésorcine et leurs sels.

Ces compositions peuvent également contenir des agents tensioactifs anioniques, cationiques, non-ioniques, amphotères ou leurs mélanges.

Parmi ces agents tensio-actifs, on peut citer les alkylbenzène sulfonates, les alkylnaphtalènesulfonates, les sulfates, les éthersulfates et les sulfonates d'alcools gras, les sels d'ammonium quaternaires tels que le bromure de triméthylcétylammonium, le bromure de cétyl pyridinium, les éthanolamides d'acides gras éventuellement oxyéthylénés, les acides, les alcools ou les amines polyoxyéthylénés, les alcools ou alphadiols polyglycérolés, les alkylphénols polyoxyéthylénés ou polyglycérolés, ainsi que les alkylsulfates polyoxyéthylénés.

Les compositions tinctoriales sont généralement aqueuses, mais elles peuvent également contenir des solvants organiques pour solubiliser des composés qui ne seraient pas suffisamment solubles dans l'eau. Parmi ces solvants, on peut citer à titre d'exemple, les alcanols inférieurs en $C_2$-$C_4$ tels que l'éthanol et l'isopropanol, le glycérol, les glycols ou éthers de glycols, comme le butoxy-2 éthanol, l'éthylèneglycol, le propylèneglycol, le monoéthyléther et le mono méthyléther du diéthylèneglycol, le monoéthyléther et le monométhyl éther du propylèneglycol, ainsi que les alcools aromatiques comme l'alcool benzylique ou le phénoxyéthanol, ou les mélanges de ces solvants.

La composition appliquée sur les cheveux peut également renfermer des agents épaississants choisis en particulier parmi l'alginate de sodium, la gomme arabique, les dérivés de cellulose tels que la méthylcellulose, l'hydroxyéthylcellulose, l'hydroxypropyl cellulose, l'hydroxyméthylcellulose, la carboxyméthylcellulose, les polymères d'acide acrylique éventuellement réticulés, la gomme de xanthane. On peut également utiliser des agents épaississants minéraux tels que la bentonite.

La composition peut également renfermer des agents antioxydants choisis en particulier parmi le sulfite de sodium, l'acide thioglycolique, le bisulfite de sodium, l'acide déhydroascorbique et l'hydroquinone, ainsi que d'autres adjuvants cosmétiquement acceptables lorsque la composition est destinée à être utilisée pour la teinture des fibres kératiniques humaines, tels que des agents de pénétration, des agents séquestrants, des conservateurs, des tampons, des parfums, etc...

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

L'invention a également pour objet la composition prête à l'emploi utilisée dans le procédé défini ci-dessus.

Selon une forme de réalisation préférée, le procédé comporte une étape préliminaire consistant à stocker sous forme séparée, d'une part, la composition contenant dans un milieu approprié pour la teinture, le coupleur répondant à la formule (I) définie ci-dessus et les précurseurs de colorants par oxydation sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus.

La composition appliquée sur les fibres kératiniques résulte d'un mélange de 10 à 90% du composant (A) avec 90 à 10% du composant (B) contenant un agent oxydant.

L'invention a également pour objet un agent de teinture des fibres kératiniques, en particulier des cheveux, essentiellement caractérisé par le fait qu'il comporte au moins deux composants, l'un des

composants étant constitué par le composant (A) défini ci-dessus et l'autre étant constitué par le composant (B) également défini ci-dessus, le pH des composants (A) et (B) étant tel qu'après mélange dans des proportions de 90 à 10% pour le composant (A) et de 10 à 90% pour le composant (B), la composition résultante ait un pH supérieur à 7.

Dans cette forme de réalisation, le composant (A) qui renferme au moins le coupleur de formule (I) et un précurseur de colorant d'oxydation, peut avoir un pH compris entre 8 et 12 et peut être ajusté à la valeur choisie au moyen d'agents alcalinisants habituellement utilisés en teinture des fibres kératiniques, tels que l'ammoniaque, les carbonates alcalins, les alcanolamines tels que les mono-, di- et triéthanolamines, ainsi que leurs dérivés ou les hydroxydes de sodium ou de potassium.

Cette composition peut renfermer les différents autres adjuvants mentionnés ci-dessus, notamment des coupleurs différents des coupleurs amino-indoliques, répondant à la formule (I) déjà mentionnée ci-dessus.

L'ensemble des précurseurs de colorants par oxydation ainsi que les coupleurs, sont présents dans des proportions comprises de préférence entre 0,05 et 7% en poids par rapport au poids total du composant (A). La concentration en composés de formule (I) peut varier entre 0,012 et 4% en poids par rapport au poids total du composant (A).

Les agents tensio-actifs sont présents dans le composant (A) dans des proportions de 0,1 à 55% en poids. Lorsque le milieu contient des solvants en plus de l'eau, ceux-ci sont présents dans des proportions comprises entre 0,5 et 40% en poids et en particulier entre 5 et 30% en poids par rapport au poids total du composant (A). Les agents épaississants sont présents de préférence dans des proportions comprises entre 0,1 et 5%, et en particulier entre 0,2 et 3% en poids. Les agents antioxydants mentionnés ci-dessus sont de préférence présents dans le composant (A) dans des proportions comprises entre 0,02 et 1,5% en poids par rapport au poids total du composant (A).

Il peut se présenter sous forme de liquide plus ou moins épaissi, de lait ou de gel.

Cet agent de teinture à deux composants peut être conditionné dans un dispositif à plusieurs compartiments ou kit de teinture, ou tout autre système de conditionnement à plusieurs compartiments dont l'un renferme le composant (A) et le second compartiment renferme le composant (B); ces dispositifs pouvant être équipés d'un moyen permettant de délivrer sur les cheveux le mélange souhaité, tel que les dispositifs décrits dans le brevet US-A-4 823 985 de la demanderesse.

L'invention a également pour objet l'utilisation comme coupleurs d'aminoindoles répondant à la formule (I) pour la teinture en milieu basique des fibres kératiniques, en association avec des précurseurs de colorants d'oxydation.

Le procédé de teinture de l'invention consiste à appliquer sur les cheveux le mélange obtenu, à le laisser poser pendant 3 à 40 minutes, puis à rincer les cheveux et éventuellement effectuer un shampooing.

Il est également possible, conformément à l'invention, d'appliquer séparément une composition contenant le coupleur de formule (I), le précurseur de colorant d'oxydation et l'agent oxydant, de façon à ce que le mélange se formant in-situ au niveau des fibres ait un pH supérieur à 7, comme défini ci-dessus.

Les exemples qui suivent sont destinés à illustrer la présente invention sans toutefois présenter un caractère limitatif.

## EXEMPLE DE PREPARATION 1

### SYNTHESE DU 4-METHYL 6-AMINOINDOLE.

1/SYNTHESE DE LA 2,3-DIMETHYL 5-NITROANILINE.

On coule 363 g de 2,3-xylidine dans 1,8 l d'acide sulfurique pur en maintenant la température à 40 °C. A cette solution refroidie à 12 °C, on ajoute un mélange sulfonitrique (132 ml d'acide nitrique (d = 1,52) et 180 ml d'acide sulfurique pur) goutte à goutte en 1 heure, la température ne dépassant pas 15 °C. Après 15 minutes, on verse sur 6 kg de glace sous agitation.

On essore le précipité beige de sulfate, lave deux fois avec 0,5 l d'eau, puis avec trois fois 0,5 l d'acétone. Le précipité est empâté avec 0,5 l d'acétone, alcalinisé avec de l'ammoniaque puis dilué avec 1,5 l d'eau glacée. Le précipité jaune est essoré, lavé à l'eau puis séché. On obtient un solide jaune possédant les caractéristiques suivantes :

Point de fusion : 110 °C.

| Analyse élémentaire pour $C_8H_{10}N_2O_2$ | | | | |
|---|---|---|---|---|
| | C | H | N | O |
| Calculé<br>Trouvé | 57,83<br>57,91 | 6,02<br>6,03 | 16,87<br>16,78 | 19,28<br>19,20 |

2/SYNTHESE DU N-(2,3-DIMETHYL 5-NITROPHENYL) FORMIMIDATE DE METHYLE.

On mélange 66,4 g de 2,3-diméthyl 5-nitroaniline, 0,4 l de triméthylorthoformiate et 1,6 g d'acide paratoluènesulfonique et on porte 3 heures au reflux.

On verse sur 1 kg de glace, on essore le précipité puis on lave avec deux fois 0,5 l d'eau. Le précipité est redissous dans 0,2 l d'acétate d'éthyle et filtré à chaud. Le solide obtenu après refroidissement est essoré, lavé à l'éther de pétrole et séché.

La cristallisation de l'éther isopropylique conduit à des cristaux blancs possédant les caractéristiques suivantes :

Point de fusion : 108 ° C.

| Analyse élémentaire pour $C_{10}H_{12}N_2O_3$ | | | | |
|---|---|---|---|---|
| | C | H | N | O |
| Calculé<br>Trouvé | 57,69<br>57,74 | 5,81<br>5,84 | 13,45<br>13,39 | 23,05<br>23,25 |

3/SYNTHESE DU 4-METHYL 6-NITROINDOLE.

A une solution de N-(2,3-diméthyl 5-nitrophényl)formimidate de méthyle (52 g) dans 0,37 l de diméthylformamide, on ajoute une solution de 31 g d'éthoxylate de potassium, 51 ml d'oxalate d'éthyle et 0,25 l de diméthylformamide. On porte la température du mélange à 40 ° C pendant 3 heures. Le précipité est essoré, lavé à l'eau. On reprend le précipité dans l'éther isopropylique à chaud puis on filtre.

Le filtrat est évaporé puis soumis à une chromatographie sur silice (éluant : heptane/acétate d'éthyle 9/1). On obtient un solide jaune possédant les caractéristiques suivantes :

Point de fusion : 145 ° C.

| Analyse élémentaire pour $C_9H_8N_2O_2$ | | | | |
|---|---|---|---|---|
| | C | H | N | O |
| Calculé<br>Trouvé | 61,36<br>61,30 | 4,58<br>4,58 | 15,90<br>15,88 | 18,16<br>18,20 |

4/SYNTHESE DU 4-METHYL 6-AMINOINDOLE.

On mélange 4,3 g de dérivé nitré préparé à l'étape 3, 20 ml d'éthanol, 9 ml de cyclohexène, 3 ml d'eau et 2,2 g de palladium sur charbon à 10%, puis on porte 2 heures au reflux.

On filtre à chaud, lave le catalyseur avec de l'alcool, évapore le filtrat sous vide. Le précipité est repris dans l'éther isopropylique, traité avec du charbon végétal, puis filtré sur célite.

Après évaporation du filtrat, on obtient un solide beige possédant les caractéristiques suivantes :

Point de fusion : 102 ° C.

| Analyse élémentaire pour $C_8H_{10}N_2$ | | | |
|---|---|---|---|
| | C | H | N |
| Calculé | 73,97 | 6,85 | 19,18 |
| Trouvé | 73,84 | 6,99 | 19,06 |

**EXEMPLE DE PREPARATION 2**

**SYNTHESE DU 4-AMINO 7-METHYLINDOLE.**

On utilise le même procédé de réduction du point 4 de l'exemple 1, en utilisant le 4-nitro 7-méthylindole à la place du 4-méthyl 6-nitro indole.

On obtient un solide jaune pâle possédant les caractéristiques suivantes :

Point de fusion : 131 ° C.

| Analyse élémentaire pour $C_8H_{10}N_2$ | | | |
|---|---|---|---|
| | C | H | N |
| Calculé | 73,97 | 6,85 | 19,18 |
| Trouvé | 73,95 | 6,94 | 19,11 |

**EXEMPLE DE PREPARATION 3**

**SYNTHESE DU 3-METHYL 6-AMINO 7-ETHYLINDOLE.**

On porte à 90 ° C un mélange de 0,5 l d'éthanol à 96 ° , 0,5 l d'acide acétique glacial et 100 g de fer pur réduit à l'hydrogène. On ajoute par portion 51 g de 3-méthyl 6-nitro 7-éthylindole en 15 minutes.

Après 2 heures à 95 ° C, on filtre les boues ferriques, le filtrat est refroidi et dilué par trois volumes d'eau. On extrait avec trois fois 0,5 l d'acétate d'éthyle, les phases organiques sont lavées, séchées et évaporées. Le résidu est repris dans l'acétate d'éthyle, traité avec du charbon végétal, filtré et refroidi.

Le précipité beige clair est essoré, lavé à l'acétate d'éthyle. Le solide est dissous dans 0,1 l d'eau, alcalinisé avec de l'ammoniaque, une huile précipite puis se solidifie. Le précipité est filtré puis lavé jusqu'à la neutralité puis séché. On obtient un solide blanc possédant les caractéristiques suivantes :

Point de fusion : 106 ° C.

| Analyse élémentaire pour $C_{11}H_{14}N_2$ | | | |
|---|---|---|---|
| | C | H | N |
| Calculé | 75,82 | 8,10 | 16,08 |
| Trouvé | 76,01 | 8,10 | 16,06 |

**EXEMPLE DE PREPARATION 4**

**SYNTHESE DU 6-AMINO 7-ETHYLINDOLE.**

A/PREPARATION DE LA 7-ETHYLINDOLINE

On prépare une solution de 145 g de 7-éthylindole dans 1,45 l de diméthoxyéthane. Sous azote, on ajoute 49,6 g de borohydrure de sodium puis on additionne sous agitation 854 g d'acide trichloroacétique en 1 heure, en maintenant la température réactionnelle à 20 ° C, à l'aide d'un bain à carboglace.

Après 2 heures d'agitation à la température ambiante, on verse sous agitation dans 2 litres de soude à 15% glacée. On ajoute 2 litres d'eau puis on extrait la phase aqueuse avec trois fois 0,4 l d'éther

isopropylique. Les phases organiques sont lavées, séchées sur sulfate de sodium puis filtrées. Le filtrat est évaporé pour conduire à 150 g d'huile (rendement quantitatif).

B/SYNTHESE DU 6-NITRO 7-ETHYLINDOLINE

a) On coule 147 g de 7-éthylindoline précédemment synthétisée dans 0,45 l d'acide sulfurique à 98% en maintenant la température à 10 °C. On mélange 42 ml d'acide nitrique à 100% et 120 ml d'acide sulfurique à 98%. On ajoute le mélange sulfonitrique goutte à goutte en 1 heure en maintenant la température de la réaction en-dessous de 5 °C. Après 1 heure à cette température, on verse sur 4 kg de glace. On neutralise à l'aide d'ammoniaque concentrée sous agitation, en contrôlant la température vers 10 °C. On extrait avec trois fois 0,4 l d'acétate d'éthyle, les phases organiques sont lavées, séchées sur sulfate de sodium, puis évaporées.
b) L'huile obtenue est dissoute dans 0,3 l d'acide chlorhydrique 12N, il y a apparition d'un précipité, celui-ci est essoré. Ce solide est lavé à l'éthanol puis à l'éther de pétrole. On reprend ce solide dans 0,3 l d'eau glacée, puis on amène la solution à pH basique à l'aide d'ammoniaque concentrée, il y a formation d'un précipité jaune; celui-ci est essoré, lavé à l'eau puis séché sous vide.
c) On dissout le solide dans 0,6 l d'éther isopropylique, on ajoute 30 g de charbon végétal CECA L2S et 10 g de sulfate de soude. Après filtration, la phase organique est évaporée à sec. L'huile orange est versée dans 0,3 l d'éther de pétrole. Le précipité orange est essoré puis séché.

On obtient 82 g de 6-nitro 7-éthylindoline.
Point de fusion : 50 °C
Rendement : 43%

C/PREPARATION DU 6-AMINO 7-ETHYLINDOLE

On dissout 38,4 g de 6-nitro 7-éthylindoline dans 120 ml d'éthanol absolu auxquels on ajoute 19,2 g de palladium sur charbon à 10% (humide à 50%), puis on porte pendant 2 heures au reflux. On filtre, on lave le catalyseur avec 100 ml d'éthanol, puis les phases organiques sont évaporées sous vide. L'extrait sec est repris dans 0,4 l d'éther isopropylique au reflux avec 3 g de charbon végétale. On filtre, sèche les phases organiques sur sulfate de sodium puis évapore à sec. Le solide est cristallisé dans 40 ml d'éther isopropylique. Les cristaux blancs sont essorés, séchés, pour obtenir :

25 g
Point de fusion : 108 °C
Rendement : 78%

**EXEMPLE DE PREPARATION 5**

**SYNTHESE DE LA 4-AMINO 7-ETHYLINDOLE**

A/SYNTHESE DE LA 4-NITRO 7-ETHYLINDOLINE

a) On coule 147 g de 7-éthylindoline précédemment synthétisée dans 0,45 l d'acide sulfurique à 98% en maintenant la température à 10 °C. On mélange 42 ml d'acide nitrique à 100% et 120 ml d'acide sulfurique à 98%. On ajoute le mélange sulfonitrique goutte à goutte en 1 heure en maintenant la température de la réaction en-dessous de 5 °C. Après 1 heure à cette température, on verse sur 4kg de glace. On neutralise à l'aide d'ammoniaque concentrée sous agitation, en contrôlant la température vers 10 °C. On extrait avec trois fois 0,4 l d'acétate d'éthyle, les phases organiques sont lavées, séchées sur sulfate de sodium, puis évaporées.
b) L'huile obtenue est dissoute dans 0,3 l d'acide chlorhydrique 12N, il y a apparition d'un précipité, celui-ci est essoré. Ce solide est lavé à l'éthanol puis à l'éther de pétrole. On reprend ce solide dans 0,3 l d'eau glacée puis on amène la solution à pH basique à l'aide d'ammoniaque concentrée, il y a formation d'un précipité jaune; celui-ci est essoré, lavé à l'eau puis séché sous vide.
c) La phase chlorhydrique obtenue en b) est diluée par 0,5 kg de glace puis amenée à pH basique à l'aide d'ammoniaque concentrée. Le précipité obtenu est essoré, lavé à l'eau, lavé avec deux fois 60 ml d'éther isopropylique et enfin séché.

Rendement : 15%
Point de fusion : 68 °C

B/PREPARATION DU 4-AMINO 7-ETHYLINDOLE

La 4-nitro 7-éthylindoline est réduite de la même manière que la 6-nitro 7-éthylindoline. On obtient 6 g d'indole recristallisé d'un mélange d'acétate d'éthyle/éther isopropylique (1/3).
Point de fusion : 123°C
Rendement : 75%

**EXEMPLE DE PREPARATION 6**

**PREPARATION DU 6(BETACHLOROETHYLURETHAN)-7-ETHYLINDOLE**

On coule 8 g de betachloroformiate dans une suspension de 8 g de 6-amino 7-éthylindole, 5,5 g de carbonate de calcium dans 24 ml de dioxane au reflux en 30 minutes. On verse sur de la glace puis on acidifie le milieu. On essore le précipité, lave à l'eau puis on le sèche. On obtient 13 g de composé blanc.
Point de fusion : 132°C
Rendement : 98%

**EXEMPLE DE PREPARATION 7**

**PREPARATION DU 3-(7-ETHYL-1H-INDOL-6-YL)-OXAZOLIDIN-2-ONE**

On porte au reflux un mélange de 8 g de l'urethane précédent, 8 ml d'éthanol à 96° et de 24 ml de soude 4N pendant 15 minutes. On verse sur glace, on essore le précipité, on lave à l'eau, à l'alcool puis à l'éther de pétrole. Après séchage, on obtient 6,6 g.
Point de fusion : 248°C
Rendement : 96%

**EXEMPLE DE PREPARATION 8**

**PREPARATION DU 7-ETHYL 6-N,β-HYDROXYETHYLAMINOINDOLE**

On ajoute 12,2 g de l'oxazolidone précédente à 50°C à un mélange de 31,5 g d'hydroxyde de potassium, 8 ml d'eau, 70 ml d'éthanol. Après 20 minutes au reflux, on verse sur 400 g de glace, on amène à pH 5 avec de l'acide acétique. On ajoute de l'ammoniaque jusqu'à pH 7,5-8. On obtient un précipité blanc. Celui-ci est lavé à l'eau et séché. L'extraction des eaux-mères donne 1,6 g supplémentaire. Le tout est cristallisé dans un mélange acétate d'éthyle/éther isopropylique (1/3). On obtient 8,9 g.
Point de fusion : 105°C
Rendement : 82%

**EXEMPLE DE TEINTURE 1**

On prépare le mélange tinctorial suivant :

| | |
|---|---|
| - 4-méthyl 6-aminoindole | 0,365 g |
| - Paraphénylènediamine | 0,27 g |
| - Nonylphénol oxyéthyléné à 4 moles d'oxyde d'éthylène, vendu sous la dénomination CEMULSOL NP 4 par la Société RHONE POULENC | 12,0 g |
| - Nonylphénol oxyéthyléné à 9 moles d'oxyde d'éthylène, vendu sous la dénomination CEMULSOL NP 9 par la Société RHONE POULENC | 15,0 g |
| - Alcool oléique polyglycérolé à 2 moles de glycérol | 1,5 g |
| - Alcool oléique polyglycérolé à 4 moles de glycérol | 1,5 g |
| - Propylèneglycol | 6,0 g |
| - Acide éthylène diamine tétracétique vendu sous la dénomination TRILON B | 0,12 g |
| - Ammoniaque à 22° Bé | 11,0 g |
| - Eau | qsp 100,0 g |
| - pH = 9,8 | |

Au moment de l'emploi, on ajoute 90 g d'eau oxygénée à 20 volumes. Le mélange dont le pH est égal à 9,7 est appliqué 30 minutes à 30°C sur cheveux naturels; il leur confère, après shampooing et rinçage, une coloration châtain cuivré.

**EXEMPLE DE TEINTURE 2**

On prépare le mélange tinctorial suivant :

| | |
|---|---|
| - 3-méthyl 7-éthyl 6-aminoindole | 0,435 g |
| - Sulfate de N,N-di(β-hydroxyéthyl)paraphénylènediamine | 0,672 g |
| - 2-butoxyéthanol | 10,0 g |
| - Alcool cétylstéarylique vendu sous la dénomination ALFOL C 16/18 par la Société CONDEA | 8,0 g |
| - Cétyl stéaryl sulfate de sodium vendu sous la dénomination CIRE DE LANETTE E par la Société HENKEL | 0,5 g |
| - Huile de ricin éthoxylée vendue sous la dénomination CEMULSOL B par la Société RHONE POULENC | 1,0 g |
| - Diéthanolamide oléique | 1,5 g |
| - Sel pentasodique de l'acide diéthylène triamine pentacétique, vendu sous la dénomination MASQUOL DTPA par la Société PROTEX | 2,5 g |
| - Ammoniaque à 22° Bé | 11,0 g |
| - Eau | qsp 100,0 g |
| - pH = 10 | |

Au moment de l'emploi, on ajoute 100 g d'eau oxygénée à 20 volumes. Le mélange dont le pH est égal à 9,7 est appliqué 25 minutes à 30°C sur cheveux naturels; il leur confère, après shampooing et rinçage, une coloration bronze gris.

## EXEMPLE DE TEINTURE 3

On prépare le mélange tinctorial suivant :

| | | |
|---|---|---|
| - 2-méthyl 5-hydroxy 6-aminoindole | | 0,405 g |
| - Paraaminophénol | | 0,272 g |
| - Alcool cétylstéarylique vendu sous la dénomination ALFOL C 16/18 par la Société CONDEA | | 19,0 g |
| - 2-octyldodécanol vendu sous la dénomination EUTANOL G par la Société HENKEL | | 4,5 g |
| - Alcool cétylstéarylique à 15 moles d'oxyde d'éthylène, vendu sous la dénomination MERGITAL C.S. par la Société HENKEL | | 2,5 g |
| - Lauryl sulfate d'ammonium | | 10,0 g |
| - Polymère cationique présentant le motif récurrent suivant : | | 4,0 g |

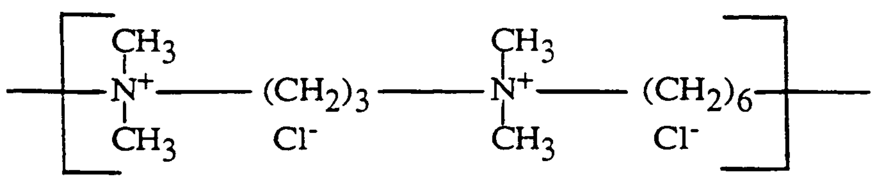

| | | |
|---|---|---|
| - Alcool benzylique | | 2,0 g |
| - Ammoniaque à 22° Bé | | 11,0 g |
| - Acide éthylène diamine tétracétique vendu sous la dénomination TRILON B | | 1,0 g |
| - Solution de bisulfite de sodium à 35° Bé | | 1,2 g |
| - Eau | qsp | 100,0 g |
| - pH = 10,3 | | |

Au moment de l'emploi, on ajoute 80 g d'eau oxygénée à 20 volumes. Le mélange dont le pH est égal à 9,4 est appliqué 20 minutes à 30 °C sur cheveux décolorés; il leur confère, après shampooing et rinçage, une coloration beige foncé.

| EXEMPLES | 4 | 5 | 6 |
|---|---|---|---|
| 7-éthyl 6-amino indole | 0,64 | 0,32 | - |
| 7-éthyl 6 N-B-hydroxyéthylaminoindole | - | - | 0,41 |
| p-phénylènediamine | - | 0,22 | 0,22 |
| p-aminophénol | 0,44 | - | - |
| Octyldodécanol vendu sous la dénomination "EUTANOL G" par HENKEL | 8 | 8 | 8 |
| Acide oléique | 20 | 20 | 20 |
| Lauryléther sulfate de mono-éthanolamine | 3 | 3 | 3 |
| Alcool éthylique | 10 | 10 | 10 |
| Alcool butylique | 10 | 10 | 10 |
| Alcool cétylstéarylique à 33 moles d'oxyde d'éthylène. | 2,4 | 2,4 | 2,4 |
| Polymère cationique constitué de motifs 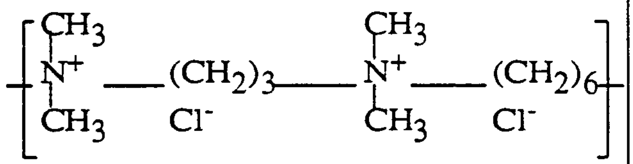 | 3,7 | 3,7 | 3,7 |
| Monoéthanolamine | 7,5 | 7,5 | 7,5 |
| Diéthanolamide d'acide linoléique | 8 | 8 | 8 |
| Ammoniaque à 20% $NH_3$ | 10,2 | 10,2 | 10,2 |
| Métabisulfite de sodium en solution aqueuse à 35% | 1,3 | 1,3 | 1,3 |
| Hydroquinone | 0,15 | 0,15 | 0,15 |
| 1-phényl 3-méthyl 5-pyrazolone | 0,2 | 0,2 | 0,2 |
| Eau déminéralisée qsp | 100 | 100 | 100 |

| EXEMPLES | 7 | 8 | 9 |
|---|---|---|---|
| 5, 7-diméthyl 6-amino indole | 0,32 | - | - |
| 5-méthyl 6-amino indole | - | 0,31 | - |
| 5, 7-diméthyl 6-amino indole | - | - | 0,33 |
| para phénylène diamine | 0,22 | 0,22 | 0,22 |
| Octyldodécanol vendu sous la dénomination "EUTANOL G" par HENKEL | 8 | 8 | 8 |
| Acide oléique | 20 | 20 | 20 |
| Lauryléther sulfate de mono-éthanolamine | 3 | 3 | 3 |
| Alcool éthylique | 10 | 10 | 10 |
| Alcool butylique | 10 | 10 | 10 |
| Alcool cétylstéarylique à 33 moles d'oxyde d'éthylène | 2,4 | 2,4 | 2,4 |
| Polymère cationique constitué de motifs 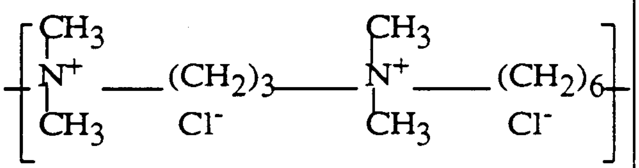 | 3,7 | 3,7 | 3,7 |
| Monoéthanolamine | 7,5 | 7,5 | 7,5 |
| Diéthanolamide d'acide linoléique | 8 | 8 | 8 |
| Ammoniaque à 20% $NH_3$ | 10,2 | 10,2 | 10,2 |
| Métabisulfite de sodium en solution aqueuse à 35% | 1,3 | 1,3 | 1,3 |
| Hydroquinone | 0,15 | 0,15 | 0,15 |
| 1-phényl 3-méthyl 5-pyrazolone | 0,2 | 0,2 | 0,2 |
| Eau déminéralisée qsp | 100 | 100 | 100 |
| pH | 10 | 10,1 | 9,9 |

Au moment de l'emploi, les compositions sont mélangées à un poids égal d'eau oxygénée à 20 volumes dont le pH est égal à 3 le pH du mélange est égal à 9,4 - 9,5.

Le mélange est appliqué pendant 30 minutes sur cheveux gris naturels. Après rinçage et lavage les cheveux sont colorés en :

Exemple 4 : blond très clair doré cuivré

Exemple 5 : blond clair cuivré

Exemple 6 : blond clair doré acajou
Exemple 7 : blond clair cuivré
Exemple 8 : blond clair cuivré légèrement acajou
Exemple 9 : bland clair cuivré

## Revendications

1. Procédé de teinture des fibres kératiniques humaines, en particulier des cheveux, caractérisé par le fait qu'on applique une composition contenant dans un milieu approprié pour la teinture, au moins un coupleur répondant à la formule :

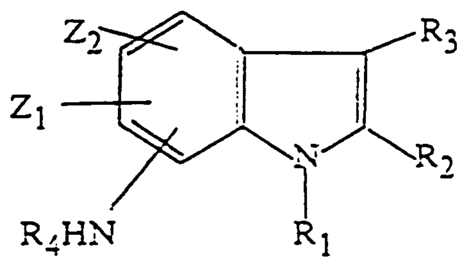

(I)

dans laquelle :

$R_1$ désigne hydrogène ou un groupement alkyle en $C_1$-$C_4$ linéaire ou ramifié;

$R_2$ et $R_3$ désignent, indépendamment l'un de l'autre, un atome d'hydrogène, un groupe alkyle en $C_1$-$C_4$ ou un groupement COOR' où R' désigne un groupe alkyle en $C_1$-$C_4$ ou un atome d'hydrogène;

au moins un des radicaux $R_2$ et $R_3$ désigne un atome d'hydrogène;

$R_4$ représente un atome d'hydrogène ou un groupement alkyle en $C_1$-$C_4$ ou un groupement hydroxyalkyle en $C_1$-$C_4$, polyhydroxyalkyle en $C_2$-$C_4$ ou aminoalkyle en $C_1$-$C_6$ dont l'amine peut éventuellement être mono- ou disubstituée par un alkyle en $C_1$-$C_4$;

le groupement $-NHR_4$ pouvant occuper les positions 4, 6 ou 7;

$Z_1$, $Z_2$, identiques ou différents, représentent un atome d'hydrogène ou un groupement alkyle en $C_1$-$C_4$, un hydroxyle, un halogène ou un alcoxy en $C_1$-$C_4$;

au moins un des groupements $Z_1$ ou $Z_2$ est différent d'hydrogène;

et les sels de tous ces composés;

- au moins un précurseur de colorant d'oxydation;
- au moins un agent oxydant;

le pH de la composition appliquée sur les fibres étant supérieur à 7.

2. Procédé selon la revendication 1, caractérisé par le fait que le composé de formule (I) est choisi parmi :

le 4-méthyl 6-aminoindole,
le 5-méthyl 6-aminoindole,
le 7-méthyl 4-aminoindole,
le 3-méthyl 7-éthyl 6-aminoindole,
le 2-méthyl 5-hydroxy 6-aminoindole,
le 5,7-diméthyl 6-aminoindole,
le 5,7-diéthyl 6-aminoindole,
le 2-éthoxycarbonyl 5-méthyl 7-aminoindole,
le 2-éthoxycarbonyl 5-chloro 7-aminioindole,
le 2-éthyoxycarbonyl 5-éthoxy 7-aminoindole,
le 2-éthoxycarbonyl 5-méthoxy 7-aminoindole,
le 5-méthoxy 7-(4'-diméthylamino 1'-méthylbutyl)aminoindole,
le 5-méthoxy 7-(4'-diméthylaminobutyl)aminoindole
le 5-méthoxy 7-(4'-diéthylamino 1'-méthylbutyl)aminoindole,
le 5-fluoro 6-aminoindole,
le 5-fluoro 1-sec-butyl 6-aminoindole,
le 5-fluoro 1-n-propyl 6-aminoindole,

le 1-méthyl 2-méthoxycarbonyl 5-méthoxy 6-aminoindole,
le 2-méthoxycarbonyl 5-méthoxy 6-aminoindole,
le 2-éthoxycarbonyl 5-méthoxy 6-aminoindole,
le 2-carboxy 5-méthoxy 6-aminoindole,
le 1,2-diméthyl 5-hydroxy 6-aminoindole,
le 2-méthoxycarbonyl 4-méthoxy 6-aminoindole,
le 7-éthyl 6-aminoindole,
le 7-éthyl 4-aminoindole,
le 7-éthyl 6-N,β-hydroxyéthylaminoindole,
et les sels de ces composés.

**3.** Procédé selon la revendication 1 ou 2, caractérisé par le fait que les précurseurs de colorants d'oxydation sont choisis parmi les paraphénylènediamines, les para-aminophénols, les précurseurs hétérocycliques para, les bases doubles.

**4.** Procédé selon la revendication 3, caractérisé par le fait que les paraphénylènediamines sont choisies parmi les composés répondant à la formule :

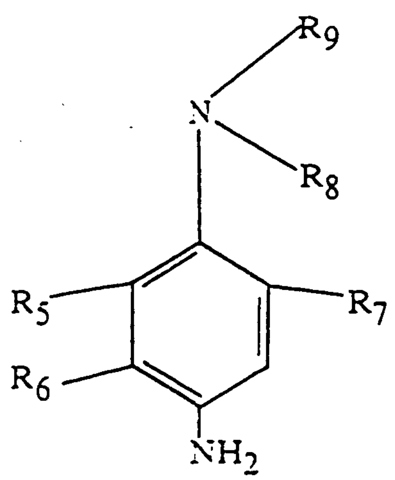

(II)

dans laquelle :

$R_5$, $R_6$ et $R_7$, identiques ou différents, représentent un atome d'hydrogène ou d'halogène, un radical alkyle ayant de 1 à 4 atomes de carbone, un radical alcoxy ayant de 1 à 4 atomes de carbone, un radical carboxy, sulfo, hydroxyalkylke ayant de 1 à 4 atomes de carbone;

$R_8$ et $R_9$, identiques ou différents, représentent un atome d'hydrogène, un radical alkyle, hydroxyalkyle, alcoxyalkyle, carbamylalkyle, aminoalkyle, mésylaminoalkyle, acétylaminoalkyle, uréidoalkyle, carbalcoxyaminoalkyle, pipéridinoalkyle, morpholinoalkyle; ces groupes alkyle ou alcoxy ayant de 1 à 4 atomes de carbone, ou bien $R_8$ et $R_9$ forment, conjointement avec l'atome d'azote auquel ils sont liés, un hétérocycle pipéridino ou morpholino, sous réserve que $R_5$ ou $R_7$ représente un atome d'hydrogène lorsque $R_8$ et $R_9$ ne représentent pas un atome d'hydrogène, ainsi que les sels de ces composés.

**5.** Procédé selon la revendication 4, caractérisé par le fait que les composés de formule (II) sont choisis parmi la p-phénylènediamine, la 2-méthyl-p-phénylènediamine, la méthoxyparaphénylènediamine, la chloroparaphénylènediamine, la 2,6-diméthylparaphénylènediamine, la 2,5-diméthylparaphénylènediamine, la 2,3-diméthylparaphénylènediamine, la 2,6-diéthylparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di-(β-hydroxyéthyl)-paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl) aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-

(éthyl,β-mésylaminoéthyl)aniline, la 3-méthyl 4-amino N,N- (éthyl,β-mésylaminoéthyl)aniline, la 4-amino N,N-(éthyl,β-sulfoéthyl) aniline, la 3-méthyl 4-amino N,N-(éthyl,β-sulfoéthyl)aniline, la N-[(4'-amino)-phényl]morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, la 2-hydroxyméthylparaphénylènediamine, la N,N-diméthyl 3-méthyl paraphénylènediamine, la N,N(éthyl,β-hydroxyéthyl)paraphénylène diamine, la N-(dihydroxypropyl)paraphénylènediamine, la N-4'-aminophényl p-phénylènediamine, la N-phényl p-phénylènediamine, sous forme de base libre ou de sels.

**6.** Procédé selon la revendication 3, caractérisé par le fait que les les p-aminophénols, sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-amino phénol, le 2-hydroxyméthyl 4-aminophénol, le 2-(β-hydroxyéthyl) 4-aminophénol, le 2-méthoxy 4-aminophénol, le 3-méthoxy 4-amino phénol, le 2,5-diméthyl 4-aminophénol, le 2-méthoxyméthyl 4-amino phénol, le 2-éthoxyméthyl 4-aminophénol, le 2-β-hydroxyéthoxyméthyl 4-aminophénol.

**7.** Procédé selon la revendication 3, caractérisé par le fait que les bases dites doubles sont choisies parmi les bis-phénylalkylène diamines de formule :

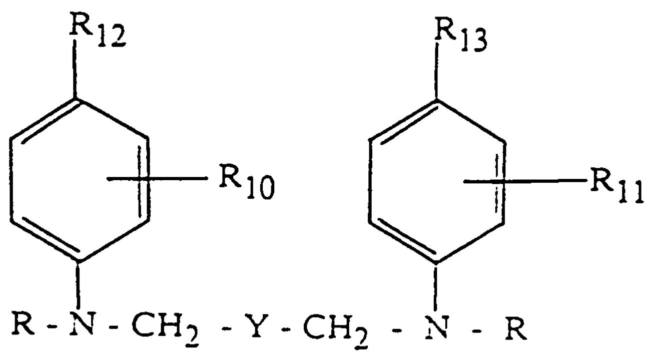

(III)

dans laquelle :

$R_{12}$ et $R_{13}$, identiques ou différents, représentent des groupements hydroxyle ou $NHR_{14}$, où $R_{14}$ désigne un atome d'hydrogène ou un radical alkyle inférieur;

$R_{10}$ et $R_{11}$, identiques ou différents, représentent soit des atomes d'hydrogène, soit des atomes d'halogène, soit des groupements alkyle;

R représente un atome d'hydrogène, un groupe alkyle, hydroxyalkyle ou aminoalkyle, dont le reste amino peut être substitué;

Y représente un radical pris dans le groupe constitué par les radicaux suivants : $-(CH_2)_n-$; $(CH_2)_{n'}-O-(CH_2)_{n'}-$;

$(CH_2)_{n'}-CHOH-(CH_2)_{n'}$ ;

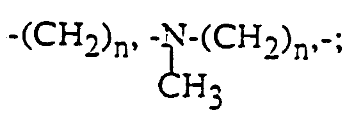

n est un nombre entier compris entre 0 et 8 et n' un nombre entier compris entre 0 et 4, ainsi que leurs sels d'addition avec des acides.

**8.** Procédé selon la revendication 7, caractérisé par le fait que la base dite double est choisie parmi les composés suivants : le N,N'bis-(β-hydroxyéthyl)N,N'-bis(4'-aminophényl)1,3-diamino 2-propanol, la N,N'bis-(β-hydroxyéthyl)N,N'-bis(4'-aminophényl)éthylènediamine, la N,N'bis-(4-aminophényl)-tétraméthylènediamine, la N,N'bis-(β-hydroxy éthyl)N,N'bis(4-aminophényl)tétraméthylènediamine, la N,N'bis-(4-méthylaminophényl)tétraméthylènediamine, la N,N'bis(éthyl)N,N'-bis (4'-amino 3'-méthylphényl)éthylènediamine.

**9.** Procédé selon la revendication 1 ou 2, caractérisé par le fait que les précurseurs de colorants d'oxydation sont des précurseurs de colorants d'oxydation de type ortho choisis parmi les orthoaminophénols et les orthophénylènediamines.

**10.** Procédé selon l'une quelconque des revendications 1 à 9, caractérisé par le fait que l'agent oxydant est choisi parmi le peroxyde d'hydrogène, le peroxyde d'urée, les bromates de métaux alcalins, les persels.

**11.** Procédé selon l'une quelconque des revendications 1 à 10, caractérisé par le fait que le pH de la composition appliquée sur les fibres kératiniques est compris entre 8 et 11.

**12.** Procédé selon l'une quelconque des revendications 1 à 11, caractérisé par le fait que la composition utilisée pour la teinture des fibres kératiniques contient en plus des coupleurs hétérocyliques de la famille des aminoindoles de formule (I), d'autres coupleurs choisis parmi les métadiphénols, les métaaminophénols, les métaphénylènediamines, les méta N-acylaminophénols, les métauréidophénols, les métacarbalcoxyaminophénols, l'$\alpha$-naphtol, les coupleurs possédant un groupement méthylène actif choisis parmi les composés dicétoniques et les pyrazolones, les coupleurs hétérocycliques ou le 4-hydroxyindole, le 6-hydroxyindole ou le 7-hydroxyindole.

**13.** Procédé selon la revendication 12, caractérisé par le fait que les coupleurs sont choisis parmi le 2,4-dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-($\beta$-hydroxyéthyl)aminophénol, le 2-méthyl 5-N-($\beta$-mésylaminoéthyl)aminophénol, la 6-hydroxybenzomorpholine, le 2,4-diaminoanisole, le 2,4-diaminophénoxyéthanol, la 6-aminobenzomorpholine, le [2-N-($\beta$-hydroxyéthyl)amino 4-amino]-phénoxyéthanol, le 2-amino 4-N-($\beta$-hydroxyéthyl)aminoanisole, le (2,4-diamino)phényl-$\beta$,$\gamma$-dihydroxypropyléther, la 2,4-diaminophénoxyéthylamine, le 1,3-diméthoxy 2,4-diaminobenzène, le 2-méthyl 5-aminophénol, le 2,6-diméthyl 3-aminophénol, le 3,4-méthylènedioxyphénol, la 3,4-méthylènedioxyaniline, la 2-chlororésorcine et leurs sels.

**14.** Procédé selon l'une quelconque des revendications 1 à 13, caractérisé par le fait que la composition contient des agents tensioactifs anioniques, cationiques, non ioniques, amphotères ou leurs mélanges; des agents épaississants, des agents anti-oxydants et/ou tout autre adjuvant cosmétiquement acceptable.

**15.** Procédé selon l'une quelconque des revendications 1 à 14, caractérisé par le fait que le milieu approprié pour la teinture est constitué par de l'eau ou par un mélange d'eau et d'un solvant choisi parmi les alcanols inférieurs en $C_2$-$C_4$, le glycérol, les glycols, les éthers de glycols, les alcools aromatiques ou leurs mélanges.

**16.** Agent de teinture des fibres kératiniques et en particulier des cheveux, caractérisé par le fait qu'il comporte au moins deux composants : un composant (A) constitué par une composition contenant dans un milieu approprié pour la teinture, un coupleur amino indolique répondant à la formule (I) tel que défini dans la revendication 1 ou 2 et un précurseur de colorant d'oxydation tel que défini dans l'une quelconque des revendications 1 et 3 à 9, un composant (B) constitué par une composition contenant dans un milieu approprié pour la teinture, un agent oxydant, le pH des composants (A) et (B) étant tel qu'après mélange dans des proportions de 90 à 10% pour le composant (A) et de 10 à 90% pour le composant (B), la composition résultante ait un pH supérieur à 7.

**17.** Agent selon la revendication 16, caractérisé par le fait que le composant (A) a un pH compris entre 8 et 12.

**18.** Agent selon la revendication 16 ou 17, caractérisé par le fait que le composant (A) contient les précurseurs de colorants par oxydation ainsi que les coupleurs dans des proportions comprises entre 0,05 et 7% en poids par rapport au poids total du composant (A).

**19.** Agent selon l'une quelconque des revendications 16 à 18, caractérisé par le fait que la concentration en composé de formule (I) est comprise entre 0,012 et 4% en poids par rapport au poids total du composant (A).

**20.** Agent selon l'une quelconque des revendications 16 à 19, caractérisé par le fait que le composant (A) contient des agents tensioactifs dans des proportions de 0,1 à 55% en poids, des solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

**21.** Procédé de teinture des fibres kératiniques et en particulier des cheveux, caractérisé par le fait qu'il comporte une première étape consistant à stocker un agent de teinture tel que défini dans l'une quelconque des revendications 16 à 21 et à procéder avant application au mélange des composants (A) et (B) dans des proportions de 10 à 90% pour le composant (A) et de 90 à 10% pour le composant (B), de façon à obtenir une composition ayant un pH supérieur à 7 et d'appliquer ce mélange immédiatement après préparation sur les fibres kératiniques.

**22.** Dispositif à plusieurs compartiments ou kit de teinture, caractérisé par le fait qu'il comporte au moins deux compartiments dont un premier compartiment renferme le composant (A) tel que défini dans l'une quelconque des revendications 16 à 20, et le second compartiment renferme le composant (B) tel que défini dans la revendication 16.

**23.** Dispositif selon la revendication 22, caractérisé par le fait qu'il est équipé d'un moyen permettant de délivrer sur les cheveux le mélange souhaité des composants (A) et (B).

**24.** Procédé de teinture selon l'une quelconque des revendications 1 à 15, caractérisé par le fait que l'on applique sur les cheveux la composition et qu'on la laisse poser pendant 3 à 40 minutes, que l'on rince les cheveux et qu'on procède éventuellement à un shampooing avant un nouveau rinçage et séchage.

**25.** Utilisation comme coupleurs des composés amino indoliques répondant à la formule (I) tels que définis dans la revendication 1 ou 2 pour la teinture en milieu basique des fibres kératiniques, en association avec des précurseurs de colorants d'oxydation.

**26.** Composition de teinture de fibres kératiniques, prête à l'emploi, telle que mise en oeuvre dans le procédé selon l'une quelconque des revendications 1 à 15.

**27.** Composition selon la revendication 26, contenant le composé de formule (I) dans des proportions de 0,01 à 3,5% en poids par rapport au poids total de la composition.

## Claims

**1.** Process for dying human keratinous fibres, in particular hair, characterised in that there is applied to these fibres a composition containing, in a suitable medium for dyeing, at least one coupler corresponding to the formula:

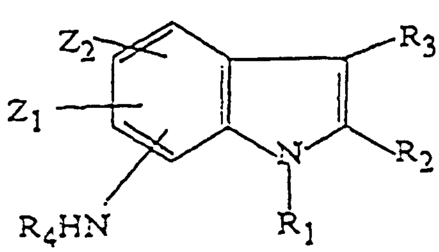

(I)

in which:

$R_1$ denotes hydrogen or a linear or branched $C_1$-$C_4$ alkyl group,

$R_2$ and $R_3$, independently of each other, denote a hydrogen atom, a $C_1$-$C_4$ alkyl group or a COOR' group, where R' denotes a $C_1$-$C_4$ alkyl radical or a hydrogen atom,

at least one of the radicals $R_2$ and $R_3$ denotes a hydrogen atom

$R_4$ denotes a hydrogen atom, a $C_1$-$C_4$ alkyl group or a $C_1$-$C_4$ hydroxyalkyl, $C_2$-$C_4$ polyhydroxyalkyl or $C_1$-$C_6$ aminoalkyl group in which the amine may be optionally mono- or disubstituted by a $C_1$-$C_4$

alkyl,

it being possible for the $-NHR_4$ group to occupy positions 4, 6 or 7,

$Z_1$ and $Z_2$, which are identical or different, denote a hydrogen atom or a $C_1$-$C_4$ alkyl, hydroxyl, halogen or $C_1$-$C_4$ alkoxy group,

at lest one of the groups $Z_1$ and $Z_2$ is other than hydrogen,

and the salts of these compounds;

- at least one oxidation dye precursor,
- at least one oxidising agent,

the pH of the composition applied to the fibres being higher than 7.

**2.** Process according to Claim 1, characterised in that the compound of formula (I) is chosen from:

4-methyl-6-aminoindole,
5-methyl-6-aminoindole,
7-methyl-4-aminoindole,
3-methyl-7-ethyl-6-aminoindole,
2-methyl-5-hydroxy-6-aminoindole,
5,7-dimethyl-6-aminoindole,
5,7-diethyl-6-aminoindole,
2-ethoxycarbonyl-5-methyl-7-aminoindole,
2-ethoxycarbonyl-5-chloro-7-aminoindole,
2-ethoxycarbonyl-5-ethoxy-7-aminoindole,
3-ethoxycarbonyl-5-methoxy-7-aminoindole,
5-methoxy-7-(4'-dimethylamino-1'-methylbutyl)aminoindole,
5-methoxy-7-(4'-dimethylaminobutyl)aminoindole,
5-methoxy-7-(4'-diethylamino-1'-methylbutyl)aminoindole,
5-fluoro-6-aminoindole,
5-fluoro-1-sec-butyl-6-aminoindole,
5-fluoro-1-n-propyl-6-aminoindole,
1-methyl-2-methoxycarbonyl-5-methoxy-6-aminoindole,
2-methoxycarbonyl-5-methoxy-6-aminoindole,
2-ethoxycarbonyl-5-methoxy-6-aminoindole,
2-carboxy-5-methoxy-6-aminoindole,
1,2-dimethyl-5-hydroxy-6-aminoindole,
2-methoxycarbonyl-4-methoxy-6-aminoindole.
7-éthyl 4-aminoindole,
7-éthyl 6-aminoindole,
7-éthyl 6-N,$\beta$-hydroxyéthylaminoindole.
and the salts of these compounds;

**3.** Process according to Claim 1 or 2, characterised in that the oxidation dye precursors are chosen from paraphenylenediamines, para-aminophenols, para-heterocyclic precursors and double bases.

**4.** Process according to Claim 3, characterised in that the para-phenylenediamines are chosen from the compounds corresponding to the formula:

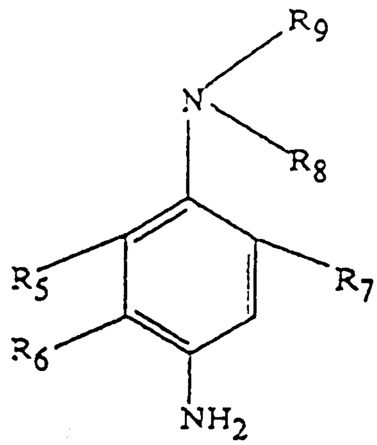

(II)

in which:

$R_5$, $R_6$ and $R_7$, which are identical or different, denote a hydrogen or halogen atom, an alkyl radical containing from 1 to 4 carbon atoms, an alkoxy radical containing from 1 to 4 carbon atoms, or a carboxyl, sulpho or hydroxyalkyl radical containing from 1 to 4 carbon atoms,

$R_8$ and $R_9$, which are identical or different, denote a hydrogen atom or an alkyl, hydroxyalkyl, alkoxyalkyl, carbamylalkyl, aminoalkyl, mesylaminoalkyl, acetylaminoalkyl, ureidoalkyl, carbalkoxyaminoalkyl, piperidinoalkyl or morpholinoalkyl radical, these alkyl or alkoxy groups containing from 1 to 4 carbon atoms, or else $R_8$ and $R_9$, together with the nitrogen atom to which they are linked, form a piperidino or morpholino heterocyclic ring, provided that $R_5$ or $R_7$ denotes a hydrogen atom when $R_8$ and $R_9$ do not denote a hydrogen atom, and the salts of these compounds.

**5.** Process according to Claim 4, characterised in that the compounds of formula (II) are chosen from p-phenylenediamine, 2-methyl-p-phenylenediamine,methoxy-para-phenylenediamine, chloro-para-phenylenediamine, 2,6-dimethyl-para-phenylenediamine, 2,5-dimethyl-para-phenylenediamine, 2,3-dimethyl-para-phenylenediamine, 2,6-diethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethyaniline, N,N-di(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl,carbamylmethyl)aniline, 3-methyl-4-amino-N,N-(ethyl,carbamylmethyl)aniline, 4-amino-N,N-(ethyl,β-piperidinoethyl)aniline, 3-methyl-4-amino-N,N-(ethyl,β-piperidinoethyl)aniline, 4-amino-N,N-(ethyl,β-morpholinoethyl)aniline, 3-methyl-4-amino-N,N-(ethyl,β-morpholinoethyl)aniline, 4-amino-N,N-(ethyl,β-acetylaminoethyl)aniline, 4-amino-N-(β-methoxyethyl)aniline, 3-methyl-4-amino-N,N-(ethyl,β-acetylaminoethyl)aniline, 4-amino-N,N-(ethyl,β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine, N-[(4'-amino)phenyl]piperidine, 2-hydroxyethyl-para-phenylenediamine,fluoro-para-phenylenediamine, carboxy-para-phenylenediamine, sulpho-para-phenylenediamine, 2-isopropyl-para-phenylenediamine, 2-n-propyl-para-phenylenediamine, 2-hydroxymethyl-para-phenylenediamine, N,N-dimethyl-3-methyl-para-phenylenediamine, N,N-(ethyl,β-hydroxyethyl)-para-phenylenediamine, N-(dihydroxypropyl)-para-phenylenediamine, N-4'-aminophenyl-p-phenylenediamine and N-phenyl-p-phenylenediamine, in the form of free base or salts.

**6.** Process according to Claim 3, characterised in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol, 2-methoxymethyl-4-aminophenol, 2-ethoxymethyl-4-aminophenol and 2-β-hydroxyethoxymethy-4-aminophenol.

**7.** Process according to Claim 3, characterised in that the so-called double bases are chosen from bis-phenylalkylenediamines of formula:

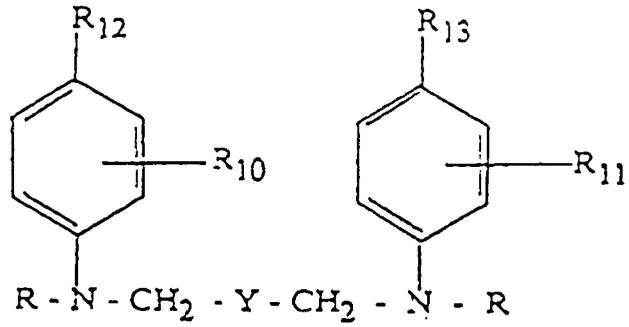

(III)

in which:

$R_{12}$ and $R_{13}$, which are identical or different, denote hydroxyl or $NHR_{14}$ groups, where $R_{14}$ denotes a hydrogen atom or a lower alkyl radical,

$R_{10}$ and $R_{11}$, which are identical or different, denote either hydrogen atoms or halogen atoms or alkyl groups,

R denotes a hydrogen atom or an alkyl, hydroxyalkyl or aminoalkyl group in which the amino residue may be substituted,

Y denotes a radical taken from the group consisting of the following radicals:
$-(CH_2)_n-$,$(CH_2)_{n'}-O-(CH_2)_{n'}-$,
$(CH_2)_{n'}-CHOH-(CH_2)_{n'}$;

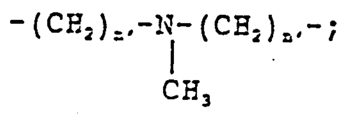

n being an integer between 0 and 8 and n' an integer between 0 and 4, and their addition salts with acids.

**8.** Process according to Claim 7, characterised in that the so-called double base is chosen from the following compounds: N,N'-bis-(β-hydroxyethyl)-N,N'-bis(4'-aminophenyl)-1,3-diamino-2-propanol, N,N'-bis(β-hydroxyethyl)-N,N'-bis(4'-aminophenyl)ethylenediamine, N,N'-bis(4-aminophenyl)-tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl)ethylenediamine.

**9.** Process according to Claim 1 or 2, characterised in that the oxidation dye precursors are oxidation dye precursors of ortho type which are chosen from ortho-aminophenols and ortho-phenylenediamines.

**10.** Process according to any one of Claims 1 to 9, characterised in that the oxidising agent is chosen from hydrogen peroxide, urea peroxide, alkali metal bromates and persalts.

**11.** Process according to any one of Claims 1 to 10, characterised in that the pH of the composition applied to the keratinous fibres is between 8 and 11.

**12.** Process according to any one of Claims 1 to 11, characterised in that the composition employed for dyeing keratinous fibres additionally contains heterocyclic couplers of the class of aminoindoles of formula (I), other couplers chosen from meta-diphenols, meta-aminophenols,meta-phenylenediamines, meta-N-acylaminophenols, meta-ureidophenols, meta-carbalkoxyaminophenols, α-naphthol, couplers containing an active methylene group and chosen from diketonic compounds and pyrazolones, heterocyclic couplers or 4-hydroxyindole, 6-hydroxyindole or 7-hydroxyindole.

**13.** Process according to Claim 12, characterised in that the couplers are chosen from 2,4-dihydroxyphenoxyethanol, 2,4-dihydroxyanisole, meta-aminophenol, resorcinol, resorcinol monomethyl ether, 2-methylresorcinol, pyrocatechol, 2-methyl-5-N-(β-hydroxyethyl)aminophenol, 2-methyl-5-N-(β-mesylaminoethyl)aminophenol, 6-hydroxybenzomorpholine, 2,4-diaminoanisole, 2,4-diaminophenoxyethanol, 6-aminobenzomorpholine, [2-N-($\beta$-hydroxyethyl)amino-4-amino]-phenoxyethanol, 2-amino-4-N-($\beta$-hydroxyethyl)aminoanisole, (2,4-diamino)phenyl-$\beta$,$\gamma$-dihydroxypropyl ether, 2,4-diaminophenoxyethylamine, 1,3-dimethoxy-2,4-diaminobenzene, 2-methyl-5-aminophenol, 2,6-dimethyl-3-aminophenol, 3,4-methylenedioxyphenol, 3,4-methylenedioxyaniline, 2-chlororesorcinol and their salts.

**14.** Process according to any one of Claims 1 to 13, characterised in that the composition contains anionic, cationic, nonionic or amphoteric surface-active agents or mixtures thereof, thickening agents, antioxidant agents and/or any other cosmetically acceptable adjuvant.

**15.** Process according to any one of Claims 1 to 14, characterised in that the suitable medium for dyeing consists of water or of a mixture of water and of a solvent chosen from $C_2$-$C_4$ lower alkanols, glycerol, glycols, glycol ethers, aromatic alcohols or their mixtures.

**16.** Agent for dyeing keratinous fibres and in particular hair, characterised in that it comprises at least two components: a component (A) consisting of a composition containing, in a suitable medium for dyeing, an aminoindole coupler corresponding to the formula (I) as defined in Claim 1 or 2 and an oxidation dye precursor as defined in any one of Claims 1 and 3 to 9, a component (B) consisting of a composition containing, in a suitable medium for dyeing, an oxidising agent, the pH of the components (A) and (B) being such that after mixing in proportions of 90 to 10% in the case of the component (A) and of 10 to 90% in the case of the component (B), the resulting composition has a pH higher than 7.

**17.** Agent according to Claim 16, characterised in that the component (A) has a pH of between 8 and 12.

**18.** Agent according to Claim 16 or 17, characterised in that the component (A) contains oxidation dye precursors and couplers in proportions of between 0.05 and 7% by weight relative to the total weight of the component (A).

**19.** Agent according to any one of Claims 16 to 18, characterised in that the concentration of compound of formula (I) is between 0.012 and 4% by weight relative to the total weight of the component (A).

**20.** Agent according to any one of Claims 16 to 19, characterised in that the component (A) contains surface-active agents in proportions of 0.1 to 55% by weight, solvents in addition to water in proportions of between 0.5 and 40% by weight, thickening agents in proportions of between 0.1 and 5% by weight, antioxidant agents in proportions of between 0.02 and 1.5% by weight, and/or any other cosmetically acceptable adjuvant.

**21.** Process for dyeing keratinous fibres and in particular hair, characterised in that it comprises a first stage consisting in storing a dyeing agent as defined in any one of Claims 16 to 20 and in mixing the components (A) and (B) before application, in proportions of 10 to 90% in the case of the component (A) and of 90 to 10% in the case of the component (B), so as to obtain a composition which has a pH higher than 7 and in applying this mixture to the keratinous fibres immediately after preparation.

**22.** Multicompartment device or dyeing kit, characterised in that it comprises at least two compartments, of which a first compartment contains the component (A) as defined in any one of Claims 16 to 20, and the second compartment contains the component (B) as defined in Claim 16.

**23.** Device according to Claim 22, characterised in that it is provided with a means permitting the desired mixture of the components (A) and (B) to be delivered onto the hair.

**24.** Dyeing process according to any one of Claims 1 to 15, characterised in that the composition is applied to the hair and is left in place for 3 to 40 minutes, that the hair is rinsed and that it is optionally shampooed before a new rinsing and drying.

**25.** Use as couplers of the aminoindole compounds corresponding to the formula (I) as defined in Claim 1 to 2 for dyeing keratinous fibres in acidic medium, in combination with oxidation dye precursors.

**26.** Composition for dyeing keratinous fibres, ready for use as used in the process according to any one of Claims 1 to 15.

**27.** Composition according to Claim 26, containing the compound of formula (I) in proportions of 0.01 to 3.5% by weight relative to the total weight of the composition.

**Patentansprüche**

**1.** Verfahren zur Färbung menschlicher keratinischer Fasern, insbesondere der Haare,
dadurch **gekennzeichnet**, daß
man eine Zusammensetzung aufbringt, die in einem zur Färbung geeigneten Medium mindestens einen Kuppler der Formel:

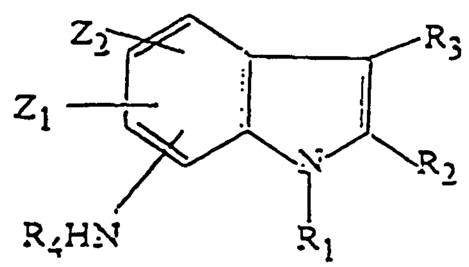

(I)

worin gilt:
$R_1$ bedeutet Wasserstoff oder eine lineare oder verzweigte $C_{1-4}$-Alkylgruppe;
$R_2$ und $R_3$ bedeuten, unabhängig voneinander, ein Wasserstoffatom, eine $C_{1-4}$-Alkylgruppe oder eine COOR'-Gruppe, worin R' eine $C_{1-4}$-Alkylgruppe oder ein Wasserstoffatom darstellt;
mindestens einer der Reste $R_2$ oder $R_3$ bedeutet ein Wasserstoffatom;
$R_4$ stellt ein Wasserstoffatom oder eine $C_{1-4}$-Alkylgruppe oder eine $C_{1-4}$-Hydroxyalkyl-, $C_{2-4}$-Polyhydroxyalkyl- oder $C_{1-6}$-Aminoalkylgruppe dar, wobei der Aminrest gegebenenfalls mit einem $C_{1-4}$-Alkylrest mono- oder disubstituiert sein kann;
die -$NHR_4$-Gruppe kann in den Positionen 4, 6 oder 7 vorliegen;
$Z_1$ und $Z_2$ stellen, gleich oder verschieden, ein Wasserstoffatom oder eine $C_{1-4}$-Alkylgruppe, einen Hydroxylrest, ein Halogenatom oder eine $C_{1-4}$-Alkoxygruppe dar;
mindestens eine der Gruppen $Z_1$ oder $Z_2$ ist verschieden von Wasserstoff;
sowie die Salze dieser Verbindungen;

- mindestens eine Oxidationsfarbstoff-Vorstufe und
- mindestens ein Oxidationsmittel enthält,

wobei der pH der auf die Fasern aufgebrachten Zusammensetzung einen Wert oberhalb 7 aufweist.

**2.** Verfahren gemäß Anspruch 1,
dadurch **gekennzeichnet**, daß
die Verbindung der Formel (I) aus:
4-Methyl-6-aminoindol,
5-Methyl-6-aminoindol,
7.Methyl-4-aminoindol,
3-Methyl-7-ethyl-6-aminoindol,
2-Methyl-5-hydrxy-6-aminoindol,
5,7-Dimethyl-6-aminoindol,
5,7-Diethyl-6-aminoindol
2-Ethoxycarbonyl-5-methyl-7-aminoindol,
2-Ethoxycarbonyl-5-chlor-7-aminoindol,
2-Ethoxycarbonyl-5-ethoxy-7-aminoindol,
2-Ethoxycarbonyl-5-methoxy-7-aminoindol,
5-Methoxy-7-(4'-dimethylamino-1'-methylbutyl)aminoindol
5-Methoxy-7-(4'-dimethylaminobutyl)aminoindol,
5-Methoxy-7-(4'-diethylamino-1'-methylbutyl)aminoindol,
5-Fluor-6-aminoindol,
5-Fluor-1-sec-butyl-6-aminoindol,
5-Fluor-1-n-propyl-6-aminoindol,

1-Methyl-2-methoxycarbonyl-5-methoxy-6-aminoindol,
2-Methoxycarbonyl-5-methoxy-6-aminoindol,
2-Ethoxycarbonyl-5-methoxy-6-aminoindol,
2-Carboxy-5-methoxy-6-aminoindol,
1,2-Dimethyl-5-hydroxy-6-aminoindol,
2-Methoxycarbonyl-4-methoxy-6-aminoindol,
7-Ethyl-6-aminoindol,
7-Ethyl-4-aminoindol,
7-Ethyl-6-N-β-hydroxyethylaminoindol
sowie aus den Salzen dieser Verbindungen ausgewählt ist.

**3.** Verfahren gemäß Anspruch 1 oder 2,
dadurch **gekennzeichnet**, daß
die Oxidationsfarbstoff-Vorstufen aus p-Phenylendiaminen, p-Aminophenolen, heterozyklischen Vorstufen vom para-Typ und aus Doppelbasen ausgewählt sind.

**4.** Verfahren gemäß Anspruch 3,
dadurch **gekennzeichnet**, daß
die p-Phenylendiamine aus Verbindungen der Formel:

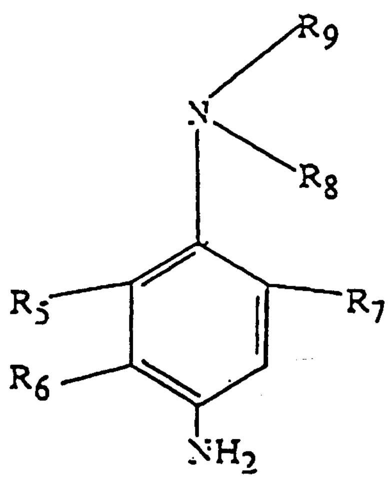

(II)

worin gilt:
$R_5$, $R_6$ und $R_7$ stellen, gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen, einen Carboxy-, Sulforest, einen Hydroxyalkylrest mit 1 bis 4 Kohlenstoffatomen dar;
$R_8$ und $R_9$ stellen, gleich oder verschieden, ein Wasserstoffatom, einen Alkyl-, Hydroxyalkyl-, Alkoxyalkyl-, Carbamylalkyl-, Aminoalkyl-, Mesylaminoalkyl-, Acetylaminoalkyl-, Ureidoalkyl-, Carbalkoxyaminoalkyl-, Piperidinoalkyl-, Morpholinoalkylrest dar, wobei die entsprechenden Alkyl- oder Alkoxygruppen 1 bis 4 Kohlenstoffatome aufweisen, oder $R_8$ und $R_9$ bilden zusammen mit dem Stickstoffatom, an das sie gebunden sind, auch einen Piperidino- oder Morpholino-Heterozyklus, mit der Maßgabe, daß $R_5$ oder $R_7$ ein Wasserstoffatom darstellen, wenn $R_8$ und $R_9$ kein Wasserstoffatom darstellen,
sowie aus den Salzen dieser Verbindungen ausgewählt sind.

**5.** Verfahren gemäß Anspruch 4,
dadurch **gekennzeichnet**, daß
die Verbindungen der Formel (II) ausgwählt sind aus p-Phenylendiamin, 2-Methyl-p-phenylendiamin, Methoxy-p-phenylendiamin, Chlor-p-phenylendiamin, 2,6-Dimethyl-p-phenylendiamin, 2,5-Dimethyl-p-phenylendiamin, 2,3-Dimethyl-p-phenylendiamin, 2,6-Diethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di-(β-hydroxyethyl)-p-phenylendiamin, 3-Methyl-4-amino-N,N-di-(β-hydroxyethyl)anilin, 3-Chlor-4-amino-N,N-di-(β-hydroxyethyl)anilin, 4-Amino-N,N-(ethyl,carbamylmethyl)-anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(methyl,β-piperidinoethyl)anilin, 4-Amino-N,N-(ethyl,β-morpholinoethyl)anilin, 3-Methyl-4-amino-N,N-(ethyl-β-morpholinoethyl)anilin, 4-Amino-N,N-(ethyl-β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin, 2-Hydroxyethyl-p-phenylendiamin, Fluor-p-phenylendiamin, Carboxy-p-phenylendiamin, Sulfo-p-phenylendiamin,2-Isopropyl-p-phenylendiamin, 2-n-Propyl-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(Dihydroxypropyl)-p-pphenylendiamin, N-4'-Aminophenyl-p-phenylendiamin, N-Phenyl-p-phenylendiamin, in Form der freien Base oder von Salzen.

6. Verfahren gemäß Anspruch 3,
dadurch **gekennzeichnet**, daß
die p-Aminophenole ausgewählt sind aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol, 2-Ethoxymethyl-4-aminophenol, 2-β-Hydroxyethoxymethyl-4-aminophenol.

7. Verfahren gemäß Anspruch 3,
dadurch **gekennzeichnet**, daß
die als Doppelbasen bezeichneten Basen aus Bisphenylalkylendiaminen der Formel:

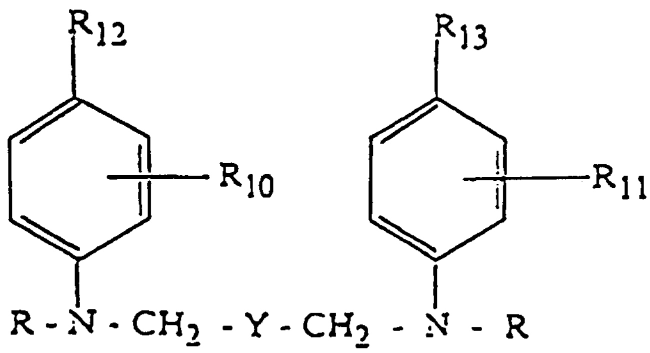

(III)

worin gilt:
$R_{12}$ und $R_{13}$ stellen, gleich oder verschieden, Hydroxyl- oder $NHR_{14}$-Gruppen dar, worin $R_{14}$ ein Wasserstoffatom oder einen Niedrigalkylrest bedeutet;
$R_{10}$ und $R_{11}$ stellen, gleich oder verschieden, entweder Wasserstoffatome oder Halogenatome oder Alkylgruppierungen dar;
R stellt ein Wasserstoffatom, eine Alkyl-, Hydroxyalkyl- oder Aminoalkylgruppe dar, deren Aminrest substituiert sein kann;
Y stellt einen Rest aus der Gruppe aus den folgenden Resten dar: $-(CH_2)_n-$; $-(CH_2)_{n'}-O-(CH_2)_{n'}-$; $-(CH_2)_{n'}-CHOH-(CH_2)_{n'}-$;

$$-(CH_2)_{n'}-\underset{\displaystyle CH3}{\underset{|}{N}}-(CH_2)_{n'}-$$

worin n eine ganze Zahl von 0 bis 8 und n' eine ganze Zahl von 0 bis 4 sind,
sowie aus deren Additionssalzen mit Säuren ausgewählt sind.

8. Verfahren gemäß Anspruch 7,
dadurch **gekennzeichnet**, daß
die als Doppelbase bezeichnete Base aus den folgenden Verbindungen ausgewählt ist: N,N'-Bis(β-hydroxyethyl)-N,N'-bis(4'-aminophenyl)-1,3-diaminopropan-2-ol, N,N'-Bis-(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis(4-aminophenyl)tetramethylendiamin, N,N'-Bis(4-methylaminophenyl)tetramethylendiamin, N,N'-Bis(ethyl)-N,N'-bis(4'-amino-3'-methylphenyl)ethylendiamin.

**9.** Verfahren gemäß Anspruch 1 oder 2,
dadurch **gekennzeichnet**, daß
die Oxidationsfarbstoff-Vorstufen Vorstufen von Oxidationsfarbstufen vom ortho-Typ sind, die aus o-Aminophenolen und o-Phenylendiaminen ausgewählt sind.

**10.** Verfahren gemäß jedem der Ansprüche 1 bis 9,
dadurch **gekennzeichnet**, daß
das Oxidationsmittel aus Wasserstoffperoxid, Harnstoffperoxid, Bromaten von Alkalimetallen, Persalzen ausgewählt ist.

**11.** Verfahren gemäß jedem der Ansprüche 1 bis 10,
dadurch **gekennzeichnet**, daß
der pH der auf die keratinischen Fasern aufgetragenen Zusammensetzung 8 bis 11 beträgt.

**12.** Verfahren gemäß jedem der Ansprüche 1 bis 11,
dadurch **gekennzeichnet**, daß
die zur Färbung der keratinischen Fasern verwendete Zusammensetzung zusätzlich zu den heterozyklischen Kupplern der Familie der Aminoindole der Formel (I) weitere Kuppler enthält, ausgewählt aus m-Diphenolen, m-Aminophenolen, m-Phenylendiaminen, m-N-Acylaminophenolen, m-Ureidophenolen, m-Carbalkoxyaminophenolen, α-Naphthol, aus Kupplern, die eine Methylengruppierung besitzen, welche aus Diketonverbindungen und Pyrazolonen, aus heterozyklischen Kupplern ausgewählt sind oder aus 4-Hydroxyindol, 6-Hydroxyindol oder 7-Hydroxyindol.

**13.** Verfahren gemäß Anspruch 12,
dadurch **gekennzeichnet**, daß
die Kuppler aus 2,4-Dihydroxyphenoxyethanol, 2,4-Dihydroxyanisol, m-Aminophenol, Resorcin, dem Monomethylether von Resorcin, 2-Methylresorcin, Pyrocatechol, 2-Methyl-5-N-(β-hydroxyethyl)-aminophenol, 2-Methyl-5-N-(β-mesylaminoethyl)aminophenol, 6-Hydroxybenzomorpholin, 2,4-Diaminoanisol, 2,4-Diaminophenoxyethanol, 6-Aminobenzomorpholin, (2-N-(β-hydroxyethyl)amino-4-amino)-phenoxyethanol, 2-Amino-4-N-(β-hydroxyethyl)aminoanisol, (2,4-Diamino)phenyl-β,γ-dihydroxypropylether, 2,4-Diaminophenoxyethylamin, 1,3-Dimethoxy-2,4-diaminobenzol, 2-Methyl-5-aminophenol, 2,6-Dimethyl-3-aminophenol, 3,4-Methylendioxyphenol, 3,4-Methylendioxyanilin, 2-Chlorresorcin und deren Salzen ausgewählt sind.

**14.** Verfahren gemäß jedem der Ansprüche 1 bis 13,
dadurch **gekennzeichnet**, daß
die Zusammensetzung anionische, kationische, nicht-ionische, amphotere oberflächenaktive Mittel oder deren Mischungen, Verdickungsmittel, Antioxidantien und/oder jeden weiteren kosmetisch zulässigen Hilfsstoff enthält.

**15.** Verfahren gemäß jedem der Ansprüche 1 bis 14,
dadurch **gekennzeichnet**, daß
das zur Färbung geeignete Medium aus Wasser oder aus einer Mischung aus Wasser und einem Lösungsmittel zusammengesetzt ist, das aus $C_{2-4}$-Niedrigalkanolen, Glycerin, Glycolen, Ethern von Glycolen, aromatischen Alkoholen oder deren Mischungen ausgewählt ist.

**16.** Mittel Zur Färbung keratinischer Fasern und insbesondere der Haare,
dadurch **gekennzeichnet**, daß
es mindestens zwei Bestandteile aufweist: einen Bestandteil (A) aus einer Zusammensetzung, die in einem zur Färbung geeigneten Medium einen im Anspruch 1 oder 2 definierten Aminoindol-Kuppler der Formel (I) sowie eine in jedem der Ansprüche 1 und 3 bis 9 definierte Oxidationsfarbstoff-Vorstufe enthält, und einen Bestandteil (B) aus einer Zusammensetzung, die in einem zur Färbung geeigneten Medium ein Oxidationsmittel enthält, wobei der pH-Wert der Bestandteile (A) und (B) so eingestellt ist, daß die Zusammensetzung, die sich nach Vermischung in Mengenanteilen von 90 bis 10% für den

Bestandteil (A) und von 10 bis 90% für den Bestandteil (B) ergibt, einen pH oberhalb 7 aufweist.

**17.** Mittel gemäß Anspruch 16,
dadurch **gekennzeichnet**, daß
der Bestandteil (A) einen pH-Wert von 8 bis 12 aufweist.

**18.** Mittel gemäß Anspruch 16 oder 17,
dadurch **gekennzeichnet**, daß
der Bestandteil (A) die Oxidationsfarbstoff-Vorstufen sowie die Kuppler in Mengenanteilen von 0,05 bis 7 Gew.% enthält, bezogen auf das Gesamtgewicht des Bestandteils (A).

**19.** Mittel gemäß jedem der Ansprüche 16 bis 18,
dadurch **gekennzeichnet**, daß
die Konzentration der Verbindung der Formel (I) 0,012 bis 4 Gew.% ausmacht, bezogen auf das Gesamtgewicht des Bestandteils (A).

**20.** Mittel gemäß jedem der Ansprüche 16 bis 19,
dadurch **gekennzeichnet**, daß
der Bestandteil (A) oberflächenaktive Mittel in Mengenanteilen von 0,1 bis 55 Gew.%, Lösungsmittel zusätzlich zum Wasser in Mengenanteilen von 0,5 bis 40 Gew.%, Verdickungsmittel in Mengenanteilen von 0,1 bis 5 Gew.%, Antioxidantien in Mengenanteilen von 0,02 bis 1,5 Gew.% und/oder jeden weiteren kosmetisch zulässigen Hilfsstoff enthält.

**21.** Verfahren zur Färbung keratinischer Fasern und insbesondere der Haare,
dadurch **gekennzeichnet**, daß
es eine erste Stufe beinhaltet, wobei man ein in jedem der Ansprüche 16 bis 21 definiertes Färbemittel vorhält und vor der Aufbringung die Bestandteile (A) und (B) in Mengenanteilen von 10 bis 90% für den Bestandteil (A) und von 90 bis 10% für den Bestandteil (B) vermischt, und zwar so, daß man eine Zusammensetzung erhält, die einen pH-Wert oberhalb 7 aufweist, und wobei man dann diese Mischung sofort nach ihrer Zubereitung auf die keratinischen Fasern aufträgt.

**22.** Vorrichtung aus mehreren Teilen oder Kit zur Färbung,
dadurch **gekennzeichnet**, daß
sie mindestens zwei Teile umfassen, deren erster Teil den in jedem der Ansprüche 16 bis 20 definierten Bestandteil (A) und deren zweiter Teil den in Anspruch 16 definierten Bestandteil (B) enthalten.

**23.** Vorrichtung gemäß Anspruch 22,
dadurch **gekennzeichnet**, daß
sie mit einem Element ausgerüstet ist, das es ermöglicht, die gewünschte Mischung aus den Bestandteilen (A) und (B) auf die Haare aufzubringen.

**24.** Färbeverfahren gemäß jedem der Ansprüche 1 bis 15,
dadurch **gekennzeichnet**, daß
man auf die Haare die Zusammensetzung aufträgt und sie 3 bis 40 Minuten lang verweilen läßt, die Haare spült und gegebenenfalls eine Schamponierung vor einer erneuten Spülung und Trocknung durchführt.

**25.** Verwendung der im Anspruch 1 oder 2 definierten Aminoiindol-Verbindungen der Formel (I), zusammen mit Oxidationsfarbstoff-Vorstufen, als Kuppler zur Färbung keratinischer Fasern in basischem Medium.

**26.** Färbezusammensetzung für keratinische Fasern, die gebrauchsfertig vorliegt, um sie im Verfahren gemäß jedem der Ansprüche 1 bis 15 zur Anwendung zu bringen.

**27.** Zusammensetzung gemäß Anspruch 26, welche die Verbindung der Formel (I) in Mengenanteilen von 0,01 bis 3,5 Gew.% enthält, bezogen auf das Gesamtgewicht der Zusammensetzung.